(19) Europäisches Patentamt
European Patent Office
Office européen des brevets

(11) EP 3 932 911 A1

(12) EUROPEAN PATENT APPLICATION
published in accordance with Art. 153(4) EPC

(43) Date of publication:
**05.01.2022 Bulletin 2022/01**

(21) Application number: **20763086.4**

(22) Date of filing: **28.02.2020**

(51) Int Cl.:
*C07D 241/36* (2006.01) *G01N 24/00* (2006.01)
*G01N 24/12* (2006.01)

(86) International application number:
**PCT/JP2020/008482**

(87) International publication number:
**WO 2020/175696 (03.09.2020 Gazette 2020/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.02.2019 JP 2019036869**

(71) Applicants:
- **Kyushu University, National University Corporation**
  **Nishi-ku**
  **Fukuoka-shi**
  **Fukuoka 819-0395 (JP)**
- **RIKEN**
  **Wako-shi**
  **Saitama 351-0198 (JP)**

(72) Inventors:
- **YANAI Nobuhiro**
  **Fukuoka-shi, Fukuoka 819-0395 (JP)**
- **KIMIZUKA Nobuo**
  **Fukuoka-shi, Fukuoka 819-0395 (JP)**
- **KOUNO Hironori**
  **Fukuoka-shi, Fukuoka 819-0395 (JP)**
- **KAWASHIMA Yusuke**
  **Fukuoka-shi, Fukuoka 819-0395 (JP)**
- **FUJIWARA Saiya**
  **Fukuoka-shi, Fukuoka 819-0395 (JP)**
- **ORIHASHI Kana**
  **Fukuoka-shi, Fukuoka 819-0395 (JP)**
- **NISHIMURA Koki**
  **Fukuoka-shi, Fukuoka 819-0395 (JP)**
- **TATEISHI Kenichiro**
  **Wako-shi, Saitama 351-0198 (JP)**
- **UESAKA Tomohiro**
  **Wako-shi, Saitama 351-0198 (JP)**

(74) Representative: **Zimmermann & Partner Patentanwälte mbB**
**Postfach 330 920**
**80069 München (DE)**

# (54) POLARIZATION SOURCE FOR DYNAMIC NUCLEAR POLARIZATION, COMPOSITION, COMPOSITION FOR DYNAMIC NUCLEAR POLARIZATION, METHOD FOR HYPERPOLARIZATION, HYPERPOLARIZED SUBSTANCE AND NMR MEASUREMENT METHOD

(57) Provided is a polarization source having high oxygen resistance and capable of stably transitioning the spin polarization of electron to nucleus even in an environment where oxygen exists. A polarization source for dynamic nuclear polarization containing a molecule represented by the following general formula. One to six of $Z^1$ to $Z^{10}$ each represent N, and the others each independently represent C-R. R represents a hydrogen atom or a substituent. n represents an integer of 1 to 4.

EP 3 932 911 A1

## Description

### Technical Field

The present invention relates to a polarization source for use for dynamic nuclear polarization of transitioning spin polarization from electrons to nuclei, a composition containing the polarization source, a hyperpolarization method using the composition, a substance hyperpolarized by the hyperpolarization method, and an NMR measurement method for such a substance.

### Background Art

When an atomic nucleus (nuclear spin) having a magnetic moment is put in a magnetostatic field, it precesses, and in that condition, when it is irradiated with an electromagnetic wave having the same frequency as that of the precessional motion, the nuclear spin resonates to provide a nuclear magnetic resonance (NMR) phenomenon to absorb the energy of the electromagnetic field. The resonant frequency in the NMR phenomenon differs depending on the nuclear species and the chemical or magnetic environment where the atomic nucleus is left, and therefore in the field of organic chemistry and biochemistry, the frequency spectrum (chemical shift value) of the NMR signal of an electric signal converted from the amount of energy absorption by the resonance is observed to analyze the molecular structure and the physical properties of a compound, which is referred to as NMR spectroscopy much employed in the art. On the other hand, in the field of medicine, a magnetic resonance imaging method (MRI) where positional information is given to the NMR signal to make a picture is employed for non-invasive examination of biological organs such as brain.

Here, when a magnetostatic field is applied to an aggregate of nuclear spins, and for example, in the case of protons, the spins cleave into an energy state facing parallel to the magnetic field and an energy state facing antiparallel to the magnetic field. Here, a value calculated by dividing the difference between the numbers of the spins (spin population) each in a different energy state by the total number of the spins is referred to as a polarization ratio, and the intensity of an NMR signal is said to be proportional to the polarization ratio. However, the polarization ratio of nuclear spins is generally a fraction of several tens of thousands at room temperature and is an extremely small value, and this is one reason of limiting the sensitivity in NMR spectroscopy and MRI.

Given the situation, as a method for hyperpolarization of nuclear spins, there is proposed a dynamic nuclear polarization method of hyperpolarizing nuclear spins by transitioning spin polarization of electrons to ambient nuclei by a solid effect or an integrated solid effect induced by electromagnetic wave irradiation. Here, as a supply source (polarization source) for electron spins, a radical and a photoexcited triplet molecule are used, and among these, a photoexcited triplet molecule takes such a state that the electron spin population is significantly biased to a specific energy state irrespective of temperature, and therefore even at room temperature, the nuclear spins can be effectively hyperpolarized as one advantage.

For example, PTL 1 proposes use of a pentacene derivative having a specific hydrocarbon group as a photoexcited triplet molecule to be such a polarization source. The publication has confirmed that dynamic nuclear polarization using the pentacene derivative as a polarization source in a sample tube gave a higher 1H spin signal as compared with samples not subjected to dynamic nuclear polarization.

### Citation List

#### Patent Literature

PTL 1: JP 2017-15443A

### Summary of Invention

#### Technical Problem

As described above, PTL 1 proposes use of a pentacene derivative as a polarization source for dynamic nuclear polarization. However, the present inventors actually evaluated the practicability of pentacene derivatives as a polarization source, and have found that, since pentacene derivatives are extremely readily oxidized and are therefore readily oxidized and decomposed in the presence of air and light, they are difficult to handle in ordinary environments. Consequently, a polarization source having high oxygen resistance needs to be newly developed in practical use for dynamic nuclear polarization in medical sites and study facilities.

Given the situation and for the purpose of solving such problems in the prior art, the present inventors have made further studies for providing a polarization source having high oxygen resistance and capable of stably transitioning

spin polarization of electrons to nuclei even in air.

Solution to Problem

As a result of assiduous studies made for solving the above-mentioned problem, the present inventors have found that an azaacene compound configured by introducing a nitrogen atom into a benzene ring that forms an acene structure can have extremely improved oxygen resistance. In addition, the inventors have further found that the azaacene compound is a photoexcited triplet molecule that forms a triplet electron by photoexcitation, like pentacene, and can function as a polarization source that supplies the triplet electron spin polarization to nuclei. The present invention has been proposed on the basis of such findings, and specifically has the following constitution.

[1] A polarization source for dynamic nuclear polarization containing a molecule represented by the following general formula (1):

General Formula (1)

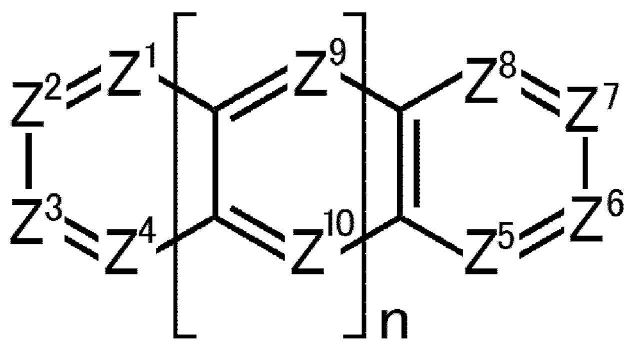

In the general formula (1), one to six of $Z^1$ to $Z^{10}$ each represent N, and the others each independently represent C-R, in which R represents a hydrogen atom or a substituent. n represents an integer of 1 to 4. When n is 2 or more, plural $Z^9$'s may be the same or different, and plural $Z^{10}$'s may be the same or different.

[2] The polarization source according to [1], wherein two to 2n (when n is 1, two) of $Z^1$ to $Z^{10}$ in the general formula (1) each represent N, and the others each independently represent C-R, in which R represents a hydrogen atom or a substituent.

[3] The polarization source according to [1] or [2], wherein n is 2 or 3.

[4] The polarization source according to any one of [1] to [3], satisfying at least one of the following three requirements:

<1> $Z^1$ and $Z^4$ are both N.
<2> $Z^5$ and $Z^8$ are both N.
<3> $Z^9$ and $Z^{10}$ are both N.

[5] The polarization source according to any one of [1] to [4], wherein at least one of plural R's in the general formula (1) is a substituent containing one or more atoms selected from the group consisting of a halogen atom, a nitrogen atom, an oxygen atom, a sulfur atom, a boron atom, a phosphorus atom and a silicon atom.

[6] The polarization source according to [5], wherein at least one of $Z^1$, $Z^2$, $Z^7$, $Z^8$ and $Z^9$ is C-$R^{11}$, at least one of $Z^3$, $Z^4$, $Z^5$, $Z^6$ and $Z^{10}$ is C-$R^{12}$, and $R^{11}$ and $R^{12}$ each are independently a substituent containing one or more atoms selected from the group consisting of a halogen atom, a nitrogen atom, an oxygen atom, a sulfur atom, a boron atom, a phosphorus atom and a silicon atom.

[7] The polarization source according to any one of [1] to [6], wherein at least one of plural R's in the general formula (1) contains one or more selected from the group consisting of a halogen atom, a cyano group, a carbonyl group, an ester group, a carboxy group, a sulfo group, a phosphono group, a phosphonoxy group, a boron atom, a phosphorus atom, a silicon atom, a hydroxy group, a thiol group, a benzene ring, a pyridine ring, a pyridazine ring, a pyrimidine ring, a pyrazine ring and an amino group.

[8] The polarization source according to any one of [1] to [7], wherein at least one of plural R's in the general formula (1) is a group containing an oligoalkyleneoxy structure.

[9] The polarization source according to any one of [1] to [8], wherein the molecule is represented by the following general formula (2):

General Formula (2)

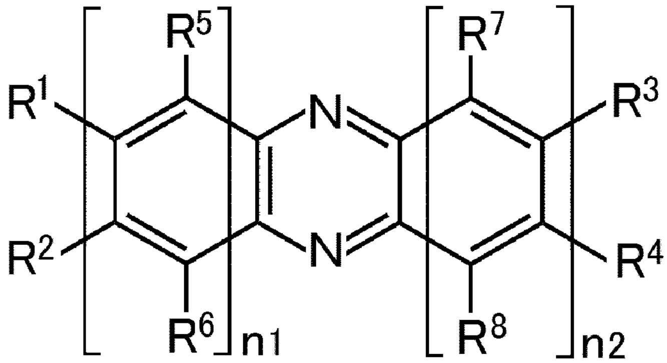

In the general formula (2), $R^1$ to $R^8$ each independently represent a hydrogen atom or a substituent. n1 and n2 each independently represent an integer of 0 to 4, and n1 + n2 is an integer of 2 to 5. When n1 is 2 or more, plural $R^5$'s may be the same or different, and plural $R^6$'s may be the same or different. When n2 is 2 or more, plural $R^7$'S may be the same or different, and plural $R^8$'s may be the same or different.

[10] A composition containing the polarization source of any one of [1] to [9].

[11] The composition according to [10], containing a porous material and the polarization source.

[12] The composition according to [11], wherein the porous material is a metal organic framework or a covalent-bonding organic framework.

[13] The composition according to [12] containing a metal organic framework, wherein the organic ligand of the metal organic framework has a cyclic structure substituted with a substituent, and at least one hydrogen atom of the substituent is substituted with a deuterium atom.

[14] The composition according to [12] containing a metal organic framework, wherein the organic ligand of the metal organic framework has an imidazole skeleton.

[15] The composition according to any one of [12] to [14] containing a metal organic framework, wherein the metal ion of the metal organic framework contains a di- to tetra-valent metal ion.

[16] The composition according to any one of [12] to [15] containing a metal organic framework, wherein the metal ion of the metal organic framework contains a zinc ion $Zn^{2+}$.

[17] The composition according to any one of [12] to [16] containing a metal organic framework, wherein the molecule constituting the polarization source has a functional group that interacts with the metal ion of the metal organic framework.

[18] The composition according to [17], wherein the functional group is an acid group.

[19] The composition according to [17], wherein the functional group is a carboxy group or a carboxylate anion.

[20] The composition according to any one of [11] to [19], wherein the molecule constituting the polarization source exists in the pores of the porous material.

[21] The composition according to any one of [11] to [20], wherein the content of the polarization source is 0.01% by mol or more relative to the number of mols of the metal ion of the metal organic framework.

[22] The composition according to any one of [11] to [21], wherein the spin-lattice relaxation time $T_1$ of the composition is 2.5 seconds or more.

[23] The composition according to any one of [10] to [22], further containing a substance capable of transitioning the nuclear spin polarization formed by the polarization source and the porous material.

[24] A composition for dynamic nuclear polarization containing the composition of any one of [10] to [23].

[25] A hyperpolarized composition prepared by hyperpolarizing the composition of any one of [10] to [23].

[26] A method for hyperpolarizing a substance, including a step of contacting a substance with the hyperpolarized composition of [25], or a step of contacting a substance with the composition of any one of [10] to [21], and then hyperpolarizing the composition to be a hyperpolarized composition.

[27] The hyperpolarization method according to [26], wherein the substance is a liquid or a solution.

[28] The hyperpolarization method according to [26], wherein the substance is a gas.

[29] The hyperpolarization method according to [27] or [28], wherein the substance is contacted with the hyperpolarized composition or the composition by infiltrating the substance into the porous material that constitutes the hyperpolarized composition or the composition.

[30] The hyperpolarization method according to any one of [26] or [29], wherein the substance contains at least one compound selected from a hydrocarbon and a derivative of a hydrocarbon in which at least one hydrogen atom is substituted with a substituent.

[31] The hyperpolarization method according to [30], wherein the substance contains a derivative of a hydrocarbon in which at least one hydrogen atom is substituted with a substituent, and at least one substituent contains an atom of which the spin quantum number I is any other than 0.

[32] The hyperpolarization method according to [30] or [31], wherein the substituent is a fluorine atom.

[33] The hyperpolarization method according to any one of [26] to [32], including a step of transitioning the nuclear spin polarization of the hyperpolarized composition to the substance.

[34] The hyperpolarization method according to [33], wherein the step of transitioning the nuclear spin polarization of the hyperpolarized compound to the substance is carried out by irradiating the hyperpolarized composition and the substance kept in contact with each other with microwaves.

[35] A substance hyperpolarized according to the method of any one of [26] to [34].

[36] An NMR measurement method including a step of measuring NMR of a substance using the composition of any one of [10] to [23].

Advantageous Effects of Invention

The molecule to be used as a polarization source in the present invention is a photoexcited triplet molecule capable of forming a triplet electron by photoexcitation, and shows high oxygen resistance. Accordingly, the structure of the polarization source of the present invention can be stably maintained even in air, and by operation of dynamic nuclear polarization, the spin polarization of the triplet electron can be stably transitioned to a nucleus.

Brief Description of Drawings

[Fig. 1] This is a schematic view for explaining a dynamic nuclear polarization mechanism with the polarization source of the present invention.

[Fig. 2] This is a graph showing time dependency of the optical density (O.D.) of a suspension of a compound 1 and that of pentacene.

[Fig. 3] This is a graph showing time dependency of the optical density (O.D.) of a solution of a compound 2 and that of tetracene.

[Fig. 4] This shows a time-resolved ESR spectrum of a p-terphenyl crystal doped with a compound 1, a compound 2 or pentacene.

[Fig. 5] This shows a temporal change of an ESR peak of a p-terphenyl crystal doped with a compound 1, a compound 2 or pentacene.

[Fig. 6] This shows a $^1H$ spin polarization buildup curve of a p-terphenyl crystal doped with a compound 1, a compound 2 or pentacene.

[Fig. 7] This is a time-resolved ESR spectrum of a compound 3 in crystalline ice.

[Fig. 8] This shows a temporal change of an ESR peak of a compound 3 in crystalline ice.

[Fig. 9] This shows a $^1H$ spin polarization buildup curve of crystalline ice containing a compound 3.

Description of Embodiments

Hereinunder the contents of the present invention are described in detail. The description of the constitutive elements given hereinunder is for some typical embodiments and specific examples of the invention, but the invention should not be limited to such embodiments and specific examples. In this description, the numerical range expressed by the wording "a number to another number" means the range that falls between the former number indicating the lower limit of the range and the latter number indicating the upper limit thereof. The hydrogen atom that is present in the molecule of a compound used in the invention is not particularly limited in isotope species, and for example, all the hydrogen atoms in the molecule may be $^1H$, and all or a part of them may be $^2H$ (deuterium (D)). In this description, "excitation light" is a light to excite a target object to cause radiation or emission, and can be a light having the same wavelength as the absorption wavelength of the target object.

<Polarization Source for Dynamic Nuclear Polarization>

The polarization source for dynamic nuclear polarization of the present invention contains a molecule represented by the general formula (1) mentioned below.

The "polarization source for dynamic nuclear polarization" in the present invention is a supply source for spin polarization to form a spin-polarized electron in dynamic nuclear polarization of hyperpolarizing a nuclear spin by transitioning spin polarization from an electron to a nucleus. Here, "polarization" or "spin polarization" means that, in Zeeman splitting having occurred by application of a magnetostatic field to a spin aggregate, the spin population at an energy level among split energy levels differs. Regarding a combination of any two of the split energy levels, a ratio of a spin population $N^1$ at one energy level to a spin population $N^2$ at the other energy level, that is, $(N_1-N_2)/(N_1+N_2)$ is referred to as a polarization ratio. Here, of all combinations of Zeeman-split energy levels, those having a polarization ratio of 0 do not undergo spin polarization, and at least one combination having an absolute value of a polarization ratio of more than 0 (positive or negative) can be said to have undergone spin polarization. A larger polarization ratio means that spin exists excessively at any one energy level to provide higher spin polarization.

In the present invention, the molecule represented by the general formula (1) is used as "a polarization source for dynamic nuclear polarization". One alone or two or more kinds of molecules represented by the general formula (1) can be used as a polarization source either singly or as combined.

The molecule represented by the general formula (1) is a photoexcited triplet molecule capable of forming a spin-polarized triplet electron through photoexcitation, and can be used as a polarization source of supplying the triplet electron spin polarization to a nucleus. The molecule represented by the general formula (1) has an azaacene skeleton configured by introducing two nitrogen atoms into one benzene ring constituting an acene structure, and is characterized by having high oxygen resistance. Consequently, the molecule is hardly oxidized even in environments in the presence of air and light, such as medical sites and study facilities, and can surely express the function as a polarization source. The reason why the molecule represented by the general formula (1) has high oxygen resistance is presumed to be because a highly electrophilic nitrogen atom is introduced into the acene structure to lower the energy level of LUMO (lowest unoccupied molecular orbital) so that electron transition from the molecule to an oxygen molecule can be suppressed.

[Molecule Represented by General Formula (1)]

The chemical structure of the molecule represented by the general formula (1) is described below. In the following description, the skeleton having a polycyclic condensed structure in the general formula (1) may be referred to as "azaacene skeleton".

General Formula (1)

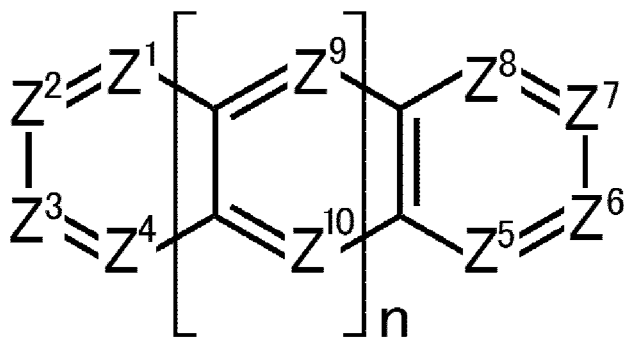

In the general formula (1), n represents an integer of 1 to 4. n is preferably 2 to 4, more preferably 2 or 3. One to six of $Z^1$ to $Z^{10}$ in the general formula (1) each represent N, and the others each independently represent C-R. When n is 1, one to six of $Z^1$ to $Z^{10}$ are N, and the other 4 to 9 each represent C-R. When n is 2, one to six of $Z^1$ to $Z^{10}$ are N, and the other 6 to 11 each represent C-R. When n is 3, one to six of $Z^1$ to $Z^{10}$ are N, and the other 8 to 13 each represent C-R. When n is 4, one to six of $Z^1$ to $Z^{10}$ are N, and the other 10 to 15 each represent C-R. Here, N means a nitrogen atom, C means a carbon atoms, and R represents a hydrogen atom or a substituent. When n is 2 or more, plural $Z^9$'s may be the same or different, and plural $Z^{10}$'s may be the same or different.

When two or more of $Z^1$ to $Z^{10}$ in the general formula (1) are N, for example, preferred examples of the case include those satisfying at least one of the following three requirements.

<1> $Z^1$ and $Z^4$ are both N.
<2> $Z^5$ and $Z^8$ are both N.
<3> $Z^9$ and $Z^{10}$ are both N.

The number of $Z^1$ to $Z^{10}$ in the general formula (1) that are N is preferably 2 to 2n. Namely, when n is 1, the number of the groups that are N is preferably 2; when n is 2, the number of the groups that are N is preferably 2 to 4;

when n is 3, the number of the groups that are N is preferably 2 to 6, and when n is 4, the number of the groups that are N is preferably 2 to 8.

Plural R's existing in the general formula (1) may be the same as or different from each other. Accordingly, plural R's may be all hydrogen atoms, or a part thereof may be hydrogen atoms and the remaining part thereof may be substituents. The substituent is preferably a substituent containing one or more atoms elected from the group consisting of a hydrogen atom, a carbon atom, a nitrogen atom, an oxygen atom, a sulfur atom, a boron atom, a phosphorus atom, a silicon atom and a halogen atom, and is more preferably a substituent formed of one or more atoms selected from the group consisting of these atoms. The number of the atoms constituting the substituent is preferably 1 to 50, more preferably 1 to 35, even more preferably 1 to 20. Examples of the substituent include a halogen atom, a cyano group, a carbonyl group, an ester group, a carboxy group, a sulfo group, a boron atom, a silicon atom, a phosphorus atom, a hydroxy group, a thiol group, a benzene ring, a pyridine ring, a pyridazine ring, a pyrimidine ring, a pyrazine ring and an amino group. Regarding the halogen atom, cyano group, carboxy group, hydroxy group, thiol group, benzene ring, pyridine ring, pyridazine ring, pyrimidine ring, pyrazine ring and amino group, the substituent may be formed of any of these groups alone.

The hydrogen atom may be $^1$H or may be $^2$H (deuterium atom). The halogen atom includes a fluorine atom, a chlorine atom, a bromine atom and an iodine tom. The amino group may be substituted or unsubstituted. Regarding acid groups such as the carboxy group, the proton of the acid group may be ionized to form an anionic group, and the anionic group may form a salt with a metal cation (e.g., sodium ion, potassium ion). Examples of the ester group include an alkyloxycarbonyl group and an aryloxycarbonyl group. Examples of the substituted amino group include a dialkylamino group, a diarylamino group, a monoalkylamine group, and a monoarylamino group. Here, the alkyl group preferably has 1 to 20 carbon atoms, more preferably 1 to 10 carbon atoms. The carbon number of the aryl group is preferably 6 to 20, more preferably 6 to 10. The alkyl group and the aryl group may be substituted, and for examples of the substituent, reference may be made to those exemplified hereinabove for the substituent for R.

One of plural R's in the general formula (1) is preferably a group containing an oligoalkyleneoxy structure. Containing an oligoalkyleneoxy structure, the dispersibility in an aqueous medium of the molecule represented by the general formula (1) extremely betters. The polarization source having high dispersibility in an aqueous medium can efficiently transition the triplet electron spin polarization thereof to the proton spin of water molecules, and therefore can be effectively used as a polarization source for NMR analysis of living bodies or biological materials. The group containing an oligoalkyleneoxy structure is preferably a group that contains an ion pair containing an ammonium ion having an oligoalkyleneoxy structure, more preferably a group that contains an ion pair of an anion group linking to C (the carbon atom constituting the ring skeleton) of C-R in the general formula (1) and an ammonium ion having an oligoalkyleneoxy structure. In other words, the molecule represented by the general formula (1) is preferably an ion pair of an anion having an azaacene skeleton in the general formula (1) and an ammonium ion having an oligoalkyleneoxy structure.

In R, the ammonium ion having an oligoalkyleneoxy structure to constitute an ion pair is preferably an ammonium ion having a group $R^{11}$-(O-$R^{12}$)n-. Here, $R^{11}$ represents a substituted or unsubstituted alkyl group, $R^{12}$ represents a substituted or unsubstituted alkylene group, and n represents an integer of 2 o 20. n $R^{12}$'s may be the same as or different from each other. The ammonium ion having an oligoalkyleneoxy structure to constitute an ion pair is more preferably an ammonium ion represented by $[R^{11}\text{-(O-}R^{12})n]_3\text{-NH}^+$. The ammonium ion represented by $[R^{11}\text{-(O-}R^{12})n]_3\text{-NH}^+$ is bulky, and therefore can further improve the dispersibility in an aqueous medium of the molecule represented by the general formula (1). Here, three $R^{11}$'s may be the same as or different from each other. 3n $R^{12}$'s may be the same as or different from each other. Three n's may be the same as or different from each other. The ammonium ion that constitutes an ion pair is especially preferably an ammonium ion represented by $[R^{11}\text{-(O-}R^{12})n]_3\text{-NH}^+$ in which the three [$R^{11}$-(O-$R^{12}$)n]'s are the same.

In each formula, the alkyl group in $R^{11}$ may be linear, branched or cyclic. The carbon number of the alkyl group is preferably 1 to 8, more preferably 1 to 4, even more preferably 1 or 2. Examples of the group include a methyl group, an ethyl group, an n-propyl group and an isopropyl group, and a methyl group is most preferred.

The alkylene group in $R^{12}$ may be linear, branched or cyclic. The carbon number of the alkylene group is preferably 1 to 8, more preferably 1 to 4, even more preferably 1 or 2. Examples of the group include a methylene group, an ethylene group and a propylene group, and an ethylene group is most preferred. Regarding the substituent that can be introduced into the alkyl group and the alkylene group, for example, reference may be made to those exemplified hereinabove for the substituent for R.

n is preferably an integer of 1 to 8, more preferably an integer of 1 to 4. For example, it may be 1 or 2, and may be selected from a range of 2 to 4.

The ammonium ion having an oligoalkyleneoxy structure may be a multimeric ammonium ion of which the structure is such that plural ammonium ions each having a group $R^{11}$-(O-$R^{12}$)n-, or an ammonium ion having a group $R^{11}$-(O-$R^{12}$)n- and any other ammonium ion bond to each other via their $R^{11}$'s, or via $R^{11}$ and a terminal of the substituent of the other ammonium ion.

The ammonium ion having an oligoalkyleneoxy structure to constitute an ion pair can be formed by acid-base

reaction between an oligoalkyleneoxy structure-having amine and a precursor of the molecule represented by the general formula (1) before forming a salt with an ammonium ion (a molecule having a proton-donating group at the position corresponding to the ion pair). Specific examples of the oligoalkyleneoxy structure-having amine for use for the formation of ammonium ion are shown below. However, the oligoalkyleneoxy structure-having amine usable in the present invention should not be limitatively interpreted by these specific examples. In the following formulae, a represents an integer of 1 to 4, m, and m1 to m3 each represents an integer of 1 to 4. m1 to m3 may be the same as or different from each other. For example, in one case, a, m and m1 to m3 are all 1. In another case, a, m, and m1 to m3 are all 2.

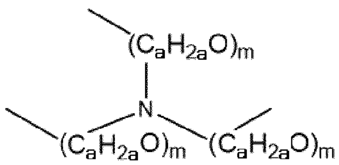
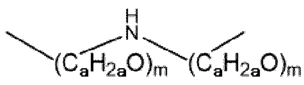
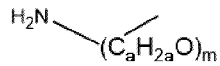

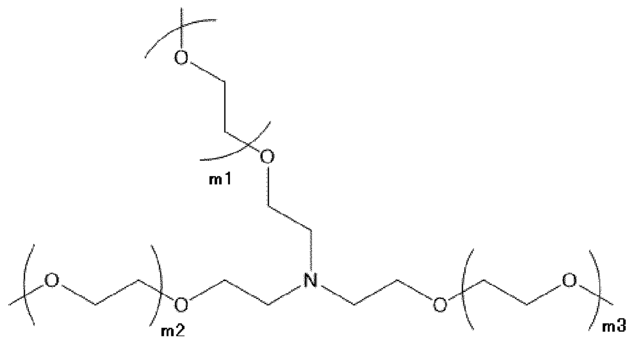
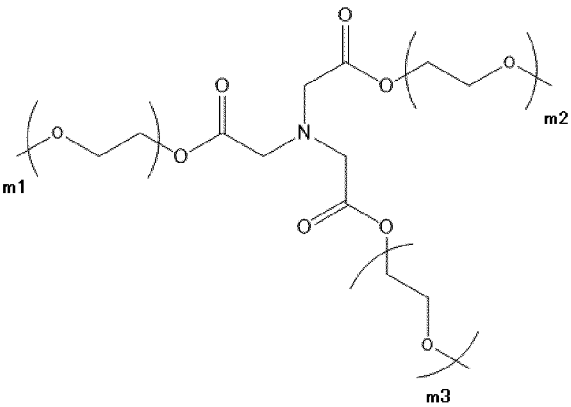

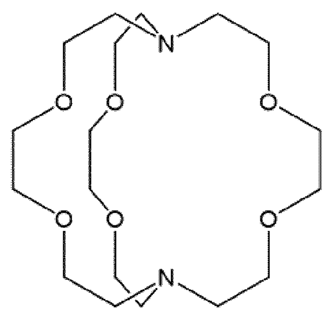

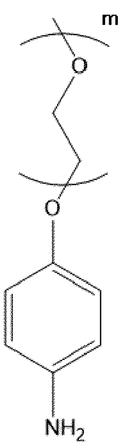
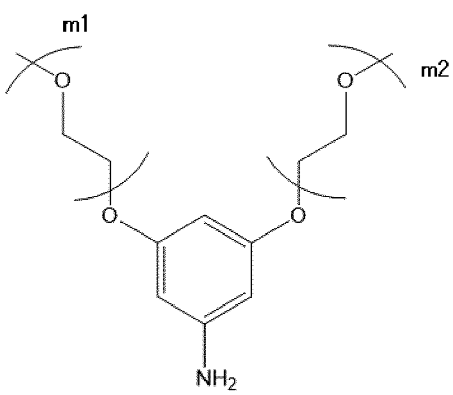
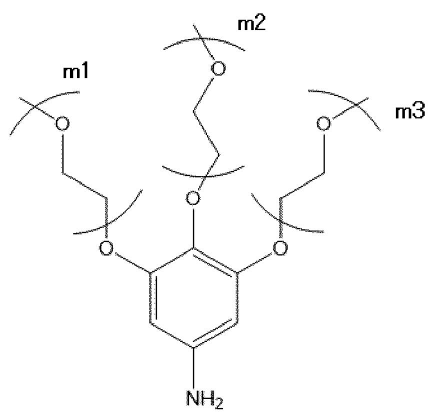

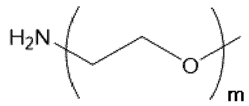
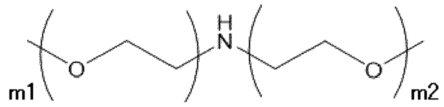
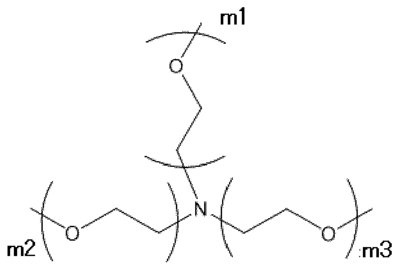
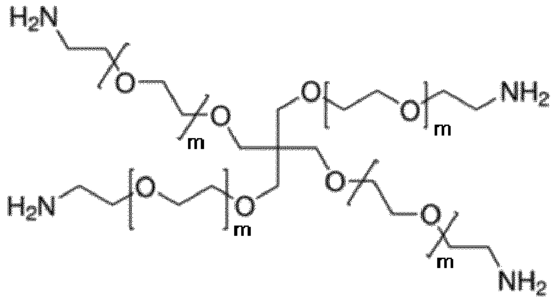
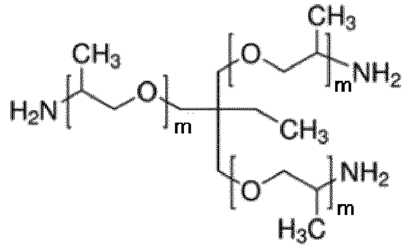

Among the ammonium ions described above, an ammonium ion represented by the following formula, which is formed by protonation of tris[2-(2-methoxyethoxy)ethyl]amine (MEEA), is most preferred.

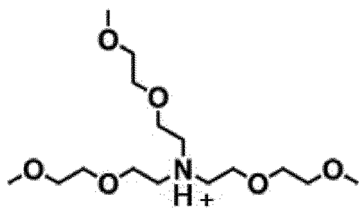

On the other hand, the anion group to constitute an ion pair with an ammonium ion having an oligoalkyleneoxy structure, that is, the anion group to bond to C (the carbon atom constituting the ring skeleton) of C-R is preferably a mono- to tetravalent anion group, and is more preferably a monovalent anion ion. Examples of the anion ion include an anion group of an acid group, such as a carboxy group (-COOH), a phosphono group ($-PO_3H_2$), a phosphonoxy group ($-OPO_3H_2$), a sulfo group ($-SO_3H$) or a sulfoxy group ($-OSO_3H$), from which the proton has been ionized. Above all, $-COO^-$, $-SO_3^-$,$-PO_3H^-$, and $-O^-$are preferred, and -COO" is more preferred.

These anion groups may be introduced into any of $Z^1$ to $Z^{10}$. Namely, C-R in which an anion group bonds to C may be any of $Z^1$ to $Z^{10}$.

The anion group may bond to C of C-R via a single bond, or via a linking group. Examples of the linking group include a substituted or unsubstituted alkylene group, and a substituted or unsubstituted arylene group. Regarding the description and preferred examples of the alkylene group, reference may be made to the description relating to the alkylene group for $R^{12}$ given hereinabove. The aromatic ring constituting the arylene group may be a single ring, or may be a condensed ring formed by condensation of two or more aromatic rings, or may be a linked ring formed by linking of two or more aromatic rings. In the case of linking two or more aromatic rings, they may link linearly or in a branched manner. The carbon number of the aromatic ring is preferably 6 to 22, more preferably 6 to 18, even more preferably 6 to 14, further more preferably 6 to 10. Specific examples of the arylene group include a phenylene group a naphthalenylene group, and a biphenylene group. Regarding the substituent that may be introduced into the alkylene group and the arylene group, for example, reference may be made to those exemplified for the substituent for R hereinabove.

The number of the anion groups to bond to the linking group may be one or may be 2 or more. In the case where 2 or more anion groups bond to the linking group, the plural anion groups may be the same as or different from each other. For example, when the linking group is a phenylene group, the number of the anion group bonding to the phenylene group is 1 to 5, and is preferably 2 to 4. The bonding position of the anion group to the phenylene group is not specifically limited, but preferably, at least one anion group bonds to the meta-position or the para-position relative to the bonding position to the azaacene skeleton, more preferably to the meta-position. When 2 or more anion groups bond to the phenylene group, preferably, at least two of them bond in the meta-position relative to each other.

In the molecule represented by the general formula (1), the number of the ion pairs of an anion group and an

ammonium ion existing in the molecule is preferably 1 or more, more preferably 2 or more, even more preferably 3 or more, further more preferably 4 or more.

In the oligoalkyleneoxy structure-containing group in R, the atomic group that contains an oligoalkyleneoxy structure may be a group (substituent) bonding to C (the carbon atom constituting the ring skeleton) of C-R via a covalent bond. Here, the "atomic group that contains an oligoalkyleneoxy structure" means a group containing an oligoalkyleneoxy structure, in which the atom-to-atom bonds are all covalent bonds (not containing an ion pair). The oligoalkyleneoxy structure-containing atomic group is preferably an atomic group having a group $R^{11}$-(O-$R^{12}$)n-. $R^{11}$ and $R^{12}$ have the same meanings as those of $R^{11}$ and $R^{12}$ of the ammonium ion having a group $R^{11}$-(O-$R^{12}$)n-mentioned hereinabove, and regarding the preferred range and specific examples thereof, reference may be made to the description relating to $R^{11}$ and $R^{12}$ given hereinabove. The group $R^{11}$-(O-$R^{12}$)n- may bond to C of C-R via a single bond, or may bond to the linking group that links to C or C-R via a covalent bond, also via a single bond.

Specific examples of the group containing an oligoalkyleneoxy group as an atomic group, which R may take, are shown below. However, the oligoalkyleneoxy structure-containing group usable in the present invention should not be limitatively interpreted by these specific examples. In the following formulae, a represents an integer of 1 to 4, and m, and m1 to m3 each represent an integer of 1 to 4. m1 to m3 may be the same as or different from each other. For example, in one case, a, m and m1 to m3 are all 1. In another case, a, m, and m1 to m3 are all 2. * indicates a bonding position to C of C-R.

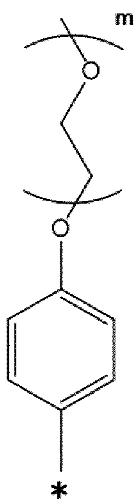

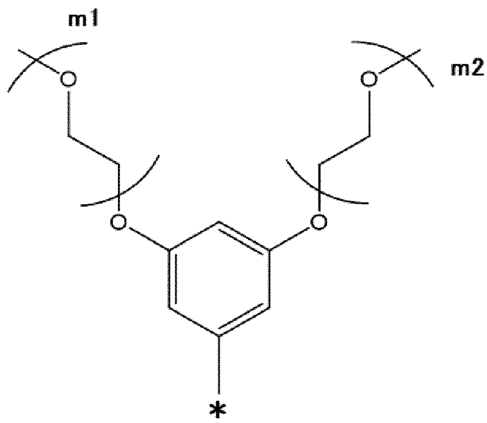

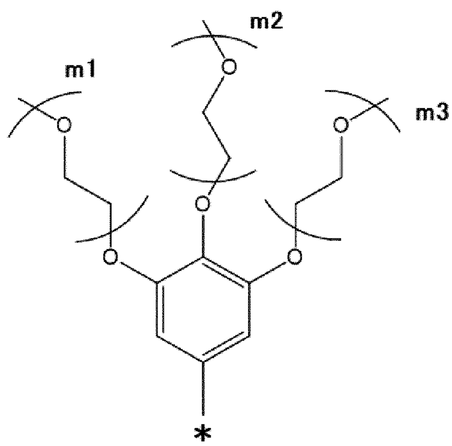

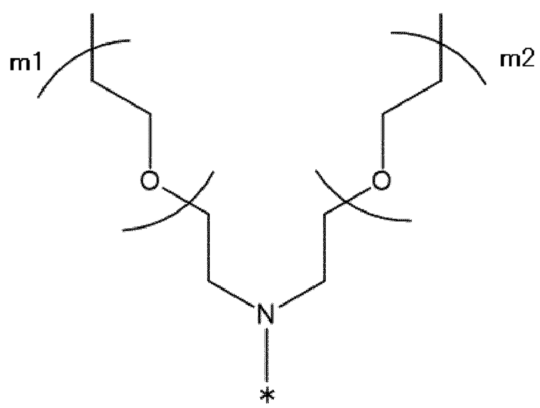

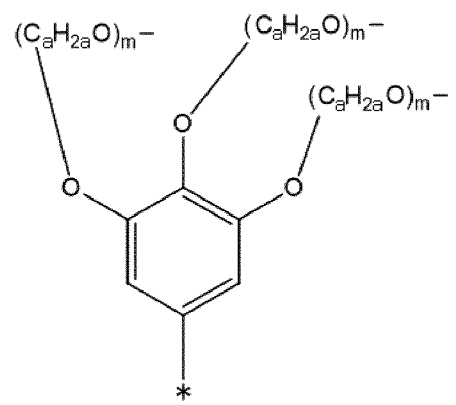

In the molecule represented by the general formula (1), the number of the oligoalkyleneoxy structures existing in the molecule is preferably 1 or more, more preferably 3 or more, even more preferably 6 or more, further more preferably 9 or more, and especially more preferably 12 or more.

The oligoalkyleneoxy structure-containing group, as well as the ion pair of an anion group and an oligoalkyleneoxy structure-having ammonium ion may be introduced into any of $Z^1$ to $Z^{10}$. Specifically, C-R in which R is an oligoalkyleneoxy structure-containing group, preferably C-R in which R is a group having an ion pair of an anion group and an oligoalkyleneoxy structure-having ammonium ion may be any of $Z^1$ to $Z^{10}$.

Also preferably, at least one of plural R's in the general formula (1) is a group having an ion pair of an anion group and an ammonium ion not having an oligoalkyleneoxy structure, and is also preferably an atomic group (substituent) not having an oligoalkyleneoxy structure. As mentioned above, when containing an oligoalkyleneoxy structure-having group in R, the dispersibility in water of the molecule represented by the general formula (1) improves. In addition, a molecule into which any other group has been introduced in place of the oligoalkyleneoxy structure-having group may also have improved dispersibility in an aqueous medium, as the case may be. Such other groups include a group having an ion pair of an anion group and a ammonium ion having an alkyl group substituted with a functional group, and a group having a substituted amino group that contains an alkyl group substituted with a functional group. Here, regarding the description and preferred examples of the alkyl group, reference may be made to the description relating to the alkyl group of $R^{11}$ given hereinabove. Examples of the functional group include a carboxy group, a hydroxy group and a carbonyl group. Regarding the description, preferred range and specific examples of the anion group, reference may be made to the description relating to the anion group of R given hereinabove.

Specific examples of amines usable in formation of an ammonium ion not having an oligoalkyleneoxy structure, and specific examples of a group having a substituted amino group as an atomic group, which may be introduced into R, are shown below. However, the amine usable in the present invention should not be limitatively interpreted by these specific examples. In the following formulae, * indicates a bonding position to C of C-R.

Specific examples of amine

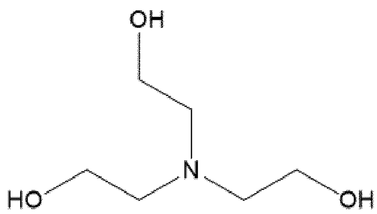
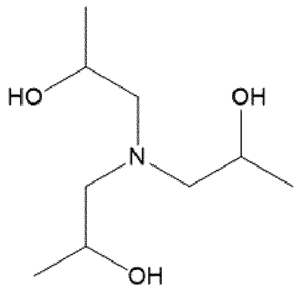

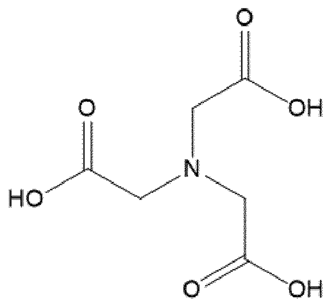
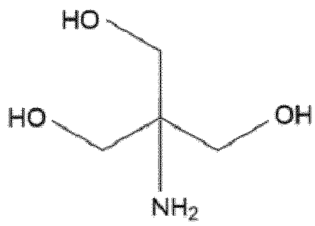

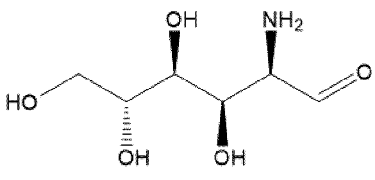
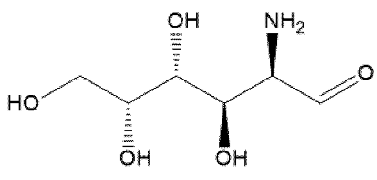
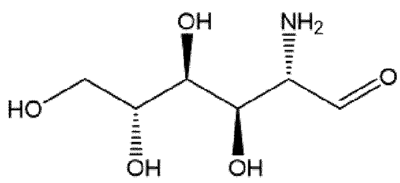

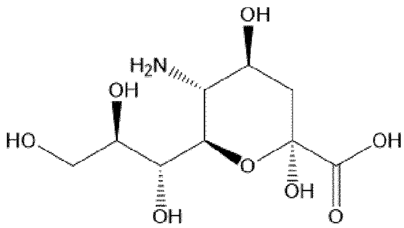

Specific examples of substituted amino group-having group

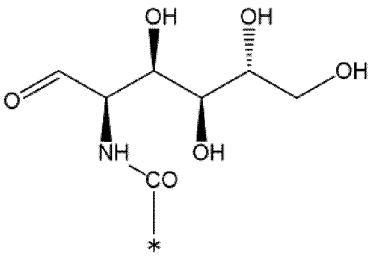

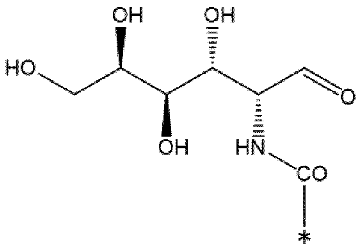

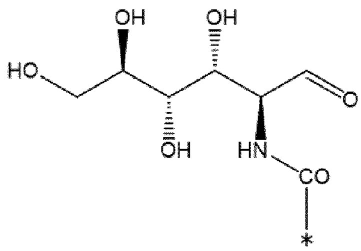

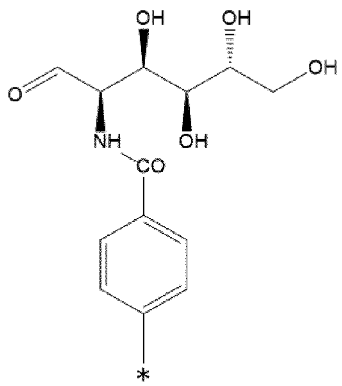

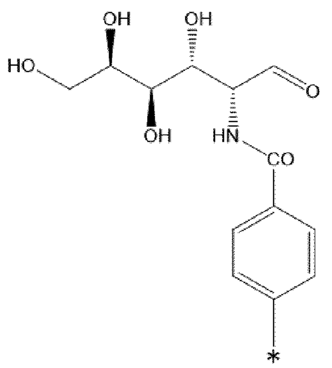

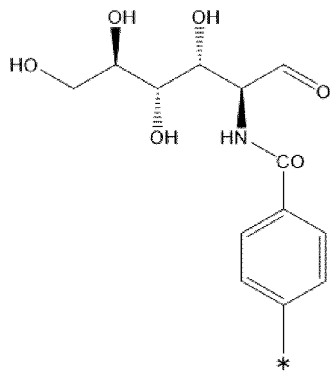

As described below, in the case where the polarization source of the present invention is combined with a porous material to constitute a composition, preferably, the azaacene skeleton in the general formula (1) is substituted with an atom or an atomic group constituting the porous material, and further when the porous material contains ions, also preferably, the skeleton is substituted with a group capable of interacting with at least any of the ions. With that, the molecules of the polarization source can be prevented from aggregating together to form an aggregate, and the molecule thereof can be readily introduced into the pores of the porous material. For example, in the case where the porous material is a metal organic framework, the molecule to be the polarization source preferably has a functional group capable of interacting with the metal ion of the metal organic framework.

In the case where the polarization source of the present invention is combined with a porous material to constitute a composition, the substituent (R) in the general formula (1) is preferably a substituent containing one or more atoms selected from a halogen atom, a nitrogen atom, an oxygen atom, a sulfur atom, a boron atom, a phosphorus atom and a silicon atom. For example, there are exemplified substituents containing one or more selected from the group consisting of a halogen atom, a cyano group, a carbonyl group, an ester group, a carboxy group, a sulfo group, a phosphono group, a phosphonoxy group, a boron atom, a phosphorus atom, a silicon atom, a hydroxy group, a thiol group, a pyridine ring, a pyridazine ring, a pyrimidine ring, a pyrazine ring and an amino group. The acid group such as a carboxy group (-COOH), a sulfo group ($-SO_3H$), a phosphono group ($-P(O)(OH)_2$) or a phosphonoxy group ($-OPO_3H_2$) may be an anion group, from which the proton has been ionized. One example thereof is a carboxylate anion group. These groups may directly bond to the azaacene skeleton, or thereto via a linking group. One preferred embodiment is a molecule of the general formula (1) where at least one of $Z^1$, $Z^2$, $Z^7$, $Z^8$ and $Z^9$ is C-$R^{11}$, at least one of $Z^3$, $Z^4$, $Z^5$, $Z^6$ and $Z^{10}$ is C-$R^{12}$, and $R^{11}$ and $R^{12}$ each are independently one or more atoms selected from the group consisting of a halogen atom, a nitrogen atom, an oxygen atom, a sulfur atom, a boron atom, a phosphorus atom and a silicon atom.

Examples of a group of the compounds included in the compound represented by the general formula (1) include a group of compounds represented by the following general formula (2).

General Formula (2)

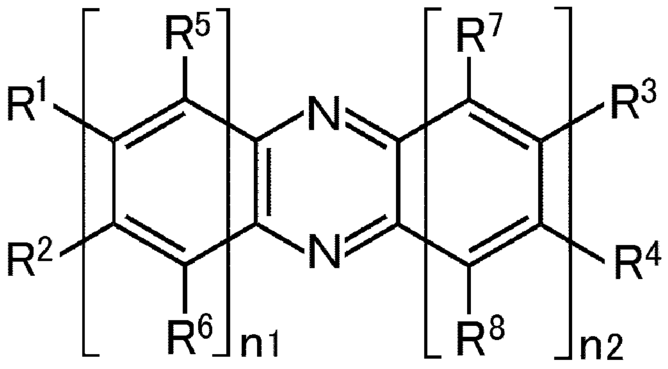

In the general formula (2), $R^1$ to $R^8$ each independently represent a hydrogen atom or a substituent. n1 and n2 each independently represent an integer of 0 to 4, and n1 + n2 is an integer of 2 to 5. When n1 is 2 or more, plural $R^5$'s may be the same or different, and plural $R^6$'s may be the same or different. When n2 is 2 or more, plural $R^7$'S may be the same or different, and plural $R^8$'s may be the same or different.

Regarding the substituents in the general formula (2), reference may be made to the description of the substituents represented by R in the general formula (1).

In the molecules represented by the general formula (1) and the general formula (2), at least a part of the hydrogen atoms may be substituted with a deuterium atom. In the case where the hydrogen atom is substituted with a deuterium atom, more preferably 30 to 70% of the hydrogen atoms are substituted with a deuterium atom. With that, the spin-lattice relaxation time for the polarization source can be prolonged to effectively enable nuclear spin hyperpolarization. Here, the site of the molecule to be substituted with a deuterium atom is preferably a relatively movable site. For example, in the case where an atomic group (substituent) bonding to the azaacene skeleton via a single bond exists in the molecule, preferably, at least a part of the hydrogen atoms that the substituent has are substituted with a deuterium atom, and preferably all the hydrogen atoms are substituted with a deuterium atom. A preferred example of the substituent to be substituted with a deuterium atom is an alkyl group having 1 to 20 carbon atoms.

Specific examples of the molecule represented by the general formula (1) are shown below. However, the molecule represented by the general formula (1) that can be used as a polarization source in the present invention should not be limitatively interpreted by these specific examples. In the following structures, "Me" represents a methyl group, and "R" represents a substituent, and is specifically a fluorine atom, a chlorine atom, a bromine atom, an iodine atom, a cyano group, a thiol group, a phenyl group, a 4-pyridyl group, a 4-methoxycarbonylphenyl group, a 4-carboxyphenyl group, a 3,5-dimethoxycarbonylphenyl group, a 3,5-dicarboxyphenyl group, a formyl group, an amino group, a dihydroxy group, $-B(OH)_2$ or

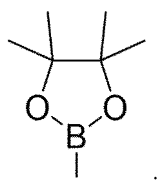

"Z" represents an ammonium ion represented by the following formula.

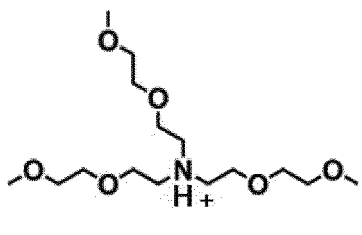

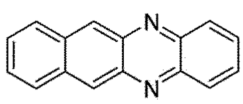
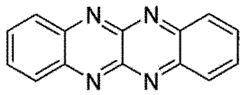
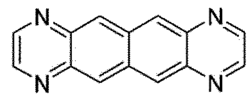

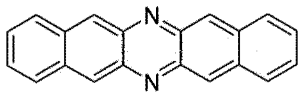
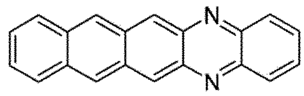
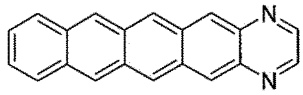

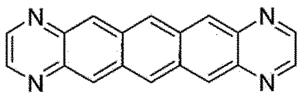
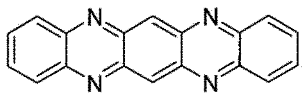
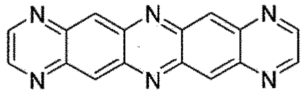

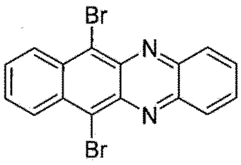
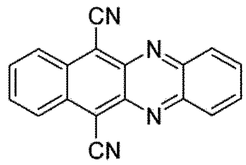
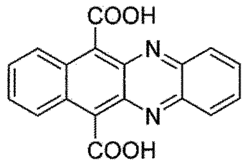
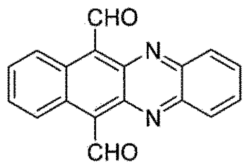

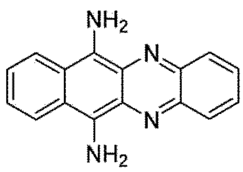
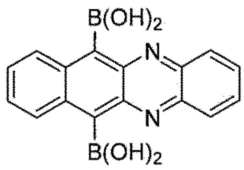
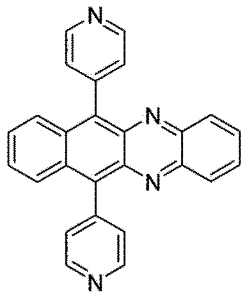
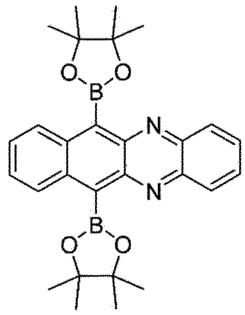

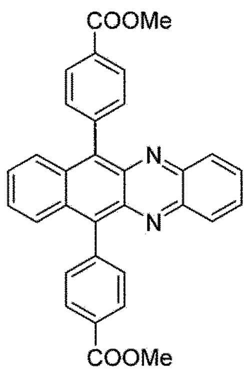
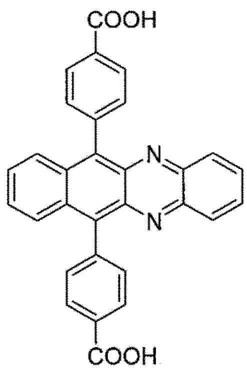
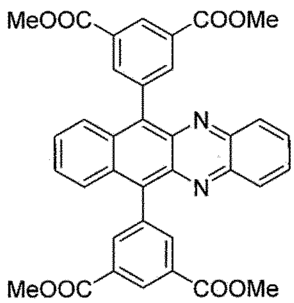
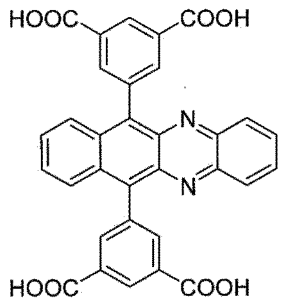

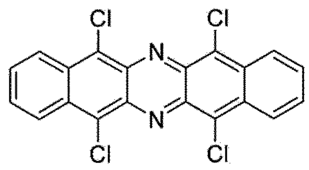
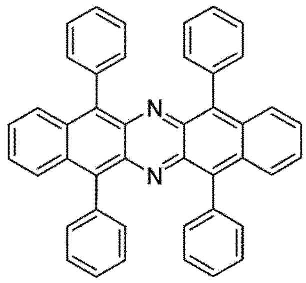
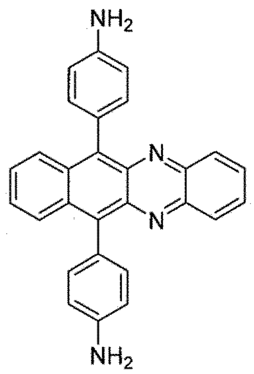
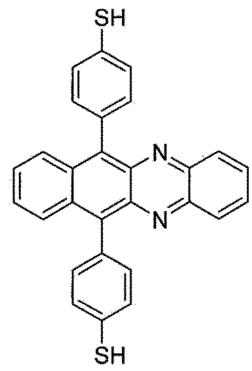

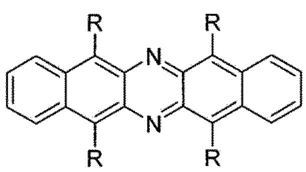
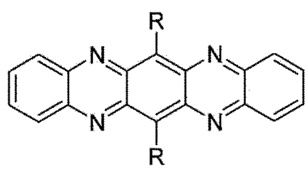
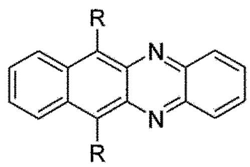

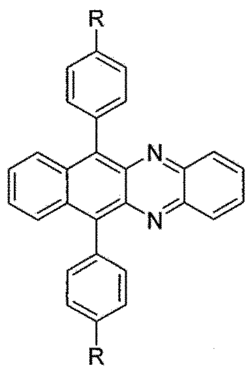
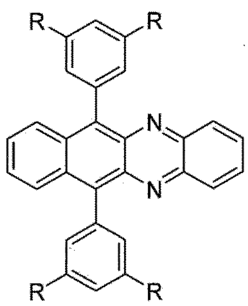
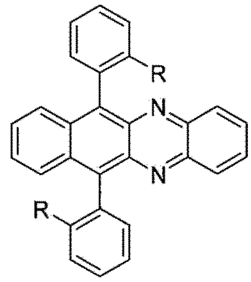
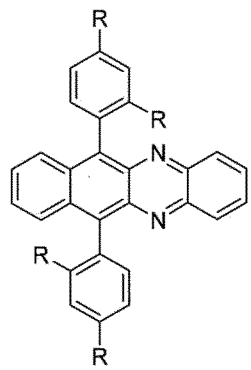

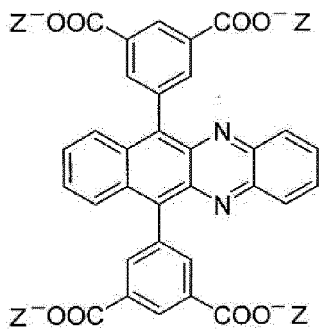
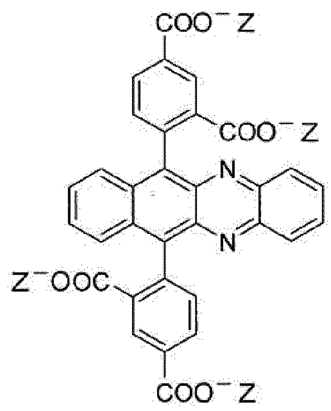

[Mechanism of Dynamic Nuclear Polarization]

In the following, a mechanism of dynamic nuclear polarization with the polarization source of the present invention is described with reference to Fig. 1. However, the mechanism of dynamic nuclear polarization with the polarization source of the present invention should not be limitatively interpreted by the following description.

[1] Polarization Source Excitation Step

In this step, the polarization source containing a molecule represented by the general formula (1) is irradiated

with excitation light to transition the molecule into an excited triplet state.

When the polarization source is irradiated with excitation light, as shown in Fig. 1, the molecule represented by the general formula (1) transitions from a ground singlet state So to an excited singlet state $S_1$, and further undergoes intersystem crossing from the excited singlet state $S_1$ to be in an excited triplet state $T_n$. Subsequently, the excited triplet state $T_n$ stepwise undergoes internal conversion into a lower-order excited triplet state, and finally into an excited triplet state $T_1$ at a lowest energy level. The number of the Zeeman level of the electron spin (triplet electron spin) in the excited triplet state $T_1$ corresponds to 3 of the magnetic quantum number m = -1, 0, +1, and is in an electron spin hyperpolarization state where the electron spins are distributed as greatly biased to a Zeeman level at m = 0 among these. On the other hand, the number of the Zeeman level of the nucleus is, for example, for a proton $^1H^+$, 2 corresponding to the magnetic quantum number m = +1/2, -1/2. Among these, at the Zeeman level at m = +1/2, the nucleus spin population is slightly larger but the polarization ratio is $10^{-6}$ or so, and is in an extremely low nucleus spin polarization state.

[2] Hyperpolarization Step

In this step, the spin polarization of the triplet electrons formed in the polarization source is transitioned to nuclear spin for nuclear spin hyperpolarization.

Specifically, for example, the polarization source having a triplet electron spin formed therein is subjected to magnetic field sweeping while irradiated with electromagnetic waves for electron spin resonance. With that, owing to an integrated solid effect, the electron spin polarization transitions to nuclei to induce nuclear spin hyperpolarization. The substance having been subjected to nuclear spin hyperpolarization in that manner (polarization target) can provide an NMR signal having a high intensity, therefore realizing high measurement sensitivity in NMR spectroscopy and MRI. In addition, since the molecule represented by the general formula (1) for use as the polarization source of the present invention has high oxygen resistance, and therefore the steps [1] and [2] can be carried out in air, and also in that case, the nuclear spin polarization can be surely improved.

Here, the hyperpolarization condition is not specifically limited. For example, the strength of the external magnetic field can be appropriately selected from a range of 0.1 to 1 T, the frequency of electromagnetic waves can be from a range of 2 to 20 GHz, and the strength of the electromagnetic field can be from a range of 0.1 to 100 W.

Regarding the above-mentioned steps [1] and [2], the step [2] can be carried out after the step [1], or the step [1] and the step [2] can be carried out simultaneously. In the latter case, the polarization source is irradiated simultaneously with excitation light and electron spin-resonating electromagnetic waves while an external magnetic field is applied thereto.

Preferably, the polarization source of the present invention is introduced into a matrix material in use thereof. In that manner, triplet electron spin polarization relaxation to be caused by molecular aggregation can be suppressed. The matrix material includes a crystal of an organic compound such as p-terphenyl, and a porous material. As it is easy to introduce the polarization source and a polarization target thereinto, a porous material is preferably used. A composition using a porous material and the polarization source of the present invention (composition of the present invention) is described below.

<Composition)>

The composition of the present invention contains a porous material and the polarization source of the present invention.

The "porous material" in the present invention is a material that is in a solid state at 20°C and 1 atmosphere, and has plural pores in the solid state. The pores that the porous material has may be through pores or non-through pores.

The composition of the present invention contains a porous material as above and the polarization source, and may include the polarization source and the polarization target inside the pores of the porous material. Having such morphology, the composition facilitates easy introduction of the polarization source and the polarization target into the inside area of the porous material, and can maintain these in a spatially dispersed state. In addition, the porous material of the type can introduce the polarization source and the polarization target as kept included inside the pores thereof, and accordingly in consideration of the introduction mode, it is not necessary to employ a soft material such as an amorphous substance and a material with emphasis on a spin-lattice relaxation time can be selected. Consequently, the composition of the present invention realizes a dynamic nuclear polarization system that has a long spin-lattice relaxation time and facilitates introduction of a polarization target thereinto.

The components that the composition of the present invention contains, and the properties of the composition are described below. Regarding the description, preferred range and specific examples of the polarization source of the present invention, reference may be made to the description of the section of <Polarization Source for Dynamic Nuclear Polarization>.

[Porous Material]

The porous material for use in the present invention includes a polarization source inside the pores thereof and functions as a substrate of maintaining the polarization source in a spatially dispersed state. Since the polarization source is maintained in a spatially dispersed state, the molecules of the polarization source can be prevented from aggregating together to relax triplet electron spin polarization. In addition, the porous material in the present invention exhibits an action of receiving, accumulating and diffusing the electron spin polarization of the polarization source in an excited triplet state at the nuclear spin. Accordingly, in the case where a polarization target is introduced into the porous material, the polarization target also receives spin polarization in the process where spin polarization has diffused inside the porous material, thereby causing nuclear spin hyperpolarization.

The porous material includes a crystalline porous polymer material such as a metal organic framework (MOF) and a covalent-bonding organic framework (COF), as well as an inorganic porous material and an organic porous material. A metal organic framework and a covalent-bonding organic framework are preferred, and a metal organic framework is more preferred. Also preferably, in the metal organic framework and the covalent-bonding organic framework, at least one hydrogen atom existing in the organic framework is substituted with a deuterium atom, and more preferably, 30 to 70% of the hydrogen atoms existing in the organic framework are substituted with a deuterium atom. With that, the composition can secure a prolonged spin-lattice relaxation time.

Here, the metal organic framework is a crystalline polymer structure in which a metal ion and a crosslinking organic ligand continuously bond to each other in a mode of coordinate bonding and which has pores inside it. The metal organic framework has a hard crystalline skeleton, and therefor secures a long sin-lattice relaxation time to thereby effectively accumulate nuclear spin polarization for hyperpolarization. In addition, in the metal organic framework, the pore size is on a nano-level and is small, and therefore, the metal organic framework can include a polarization source and a polarization target each in the form of individual molecules or as a small number of molecules inside the pores thereof and can suppress aggregation of the molecules. Further, the metal organic framework has properties of both a metal and an organic ligand, and therefore can vary the properties thereof in various ways. Accordingly, for example, by controlling the interaction between the metal organic framework and the polarization source or the polarization target, the introduction amount and the hyperpolarization state can be optimized.

The metal ion to constitute the metal organic framework is not specifically limited and is preferably a metal ion of transition metals or typical metals of Groups 2, 13 and 14. More preferred are ions of copper, zinc, cadmium, silver, cobalt, nickel, iron, ruthenium, aluminum, chromium, molybdenum, manganese, palladium, rhodium, zirconium, titanium, magnesium and lanthanum; even more preferred are ions of zinc, aluminum, zirconium and lanthanum; and especially preferred are a zinc ion and a zirconium ion. One alone or two or more kinds of these ions can be used either singly or as combined.

The valence of the metal ion is not specifically limited, but is preferably 2 to 6-valent, more preferably 2 to 5-valent, even more preferably 2 to 4-valent.

The crosslinking organic ligand is a coordinating compound having at least 2 coordinating groups. The plural coordinating groups that the organic ligand has may be the same as or different from each other. The number of the coordinating groups that the organic ligand has is preferably 2 to 8, more preferably 2 to 6, even more preferably 2 to 4. Specific examples of the coordinating group include a coordinating nitrogen atom that a nitrogen atom-containing hetero ring contains as a ring member, and a carboxy group. Among these, the carboxy group functions as a coordinating group in the form of a carboxylate anion from in a proton has been ionized.

The coordinating nitrogen atom-containing hetero ring may be an aliphatic hetero ring or an aromatic hetero ring. The coordinating nitrogen atom-containing hetero ring includes an imidazole ring, a triazine ring, a pyridine ring, and a pyrimidine ring, and an imidazole ring is preferred. These hetero rings may be substituted with a substituent. Regarding the preferred range and specific examples of the substituent, reference may be made to the preferred range and specific examples of $R^1$ to $R^3$ mentioned below.

Not specifically limited, the organic structure substituted with a carboxy group to be a coordinating group includes an aromatic hydrocarbon ring, an alkene, a hetero ring containing the above-mentioned coordinating nitrogen atom, an alkane, an alkyne, and a non-aromatic hydrocarbon ring. These organic structures may be substituted with a substituent. Regarding the preferred range and specific examples of the substituent, reference may be made to the preferred range and specific examples of the substituent for $R^1$ to $R^3$ mentioned below.

In the case where the organic ligand of a metal organic framework has a cyclic structure substituted with a substituent, at least one hydrogen atom that the substituent contains is preferably substituted with a deuterium atom, and preferably, all the hydrogen atoms of the substituent are substituted with deuterium atoms.

More preferred examples of the crosslinking organic ligand include an imidazole ligand represented by the following general formula (B).

General Formula (B)

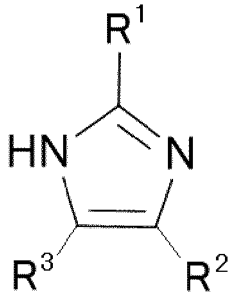

In the general formula (B), $R^1$ to $R^3$ each independently represent a hydrogen atom or a substituent. When two or more of $R^1$ to $R^3$ are substituents, the substituents may be the same as or different from each other. Preferably, the substituent of $R^1$ to $R^3$ is such that at least a part of the hydrogen atoms that the substituent contains are substituted with deuterium atoms, and preferably, all the hydrogen atoms that the substituent contains are substituted with deuterium atoms. Having the constitution, the composition can secure a prolonged spin-lattice relaxation time. The number of the substituents of $R^1$ to $R^3$ is not specifically limited. Preferably, at least $R^1$ is a substituent. The substituent represented by $R^1$ is preferably a substituted or unsubstituted alkyl group, more preferably an alkyl group in which at least a part of the hydrogen atoms are substituted with a deuterium atom, even more preferably an alkyl group in which all hydrogen atoms are substituted with deuterium atoms. The carbon number of the alkyl group is preferably 1 to 20, more preferably 1 to 4, even more preferably 1.

Examples of the substituent that $R^1$ to $R^3$ may take include a hydroxy group, a halogen atom, an alkyl group having 1 to 20 carbon atoms, an alkoxy group having 1 to 20 carbon atoms, an alkylthio group having 1 to 20 carbon atoms, an alkyl-substituted amino group having 1 to 20 carbon atoms, an aryl-substituted amino group having 1 to 20 carbon atoms, an aryl group having 6 to 40 carbon atoms, a heteroaryl group having 3 to 40 carbon atoms, an alkenyl group having 2 to 10 carbon atoms, an alkynyl group having 2 to 10 carbon atoms, an alkylamide group having 2 to 20 carbon atoms, an arylamide group having 7 to 21 carbon atoms, a trialkylsilyl group having 3 to 20 carbon atoms, and an oligoethylene glycol group having 2 to 20 carbon atoms. Among these specific examples, those further substitutable with any substituent may be substituted.

Specific examples of the crosslinking organic ligand include those of the following formulae. In the following organic ligands of which the ring structure is substituted with a methyl group, the hydrogen atoms of the methyl group may be substituted with deuterium atoms, and these are also specific examples of the crosslinking organic ligand. However, the organic ligand of the metal organic framework usable as a porous material in the present invention should not be limitatively interpreted by these specific examples.

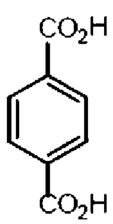

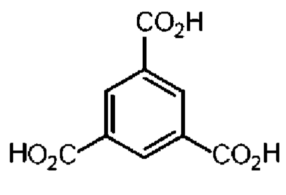

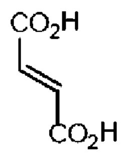

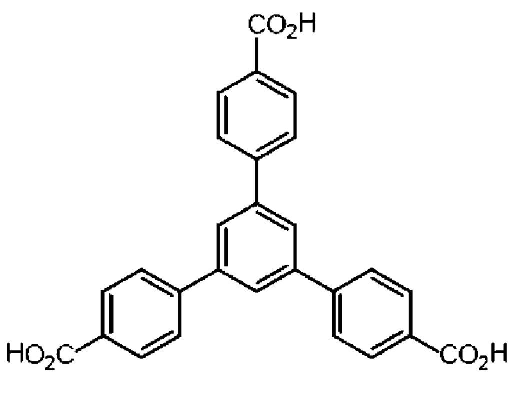

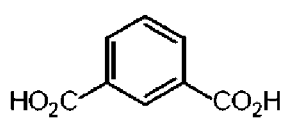

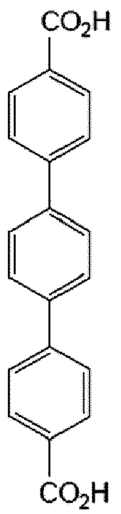
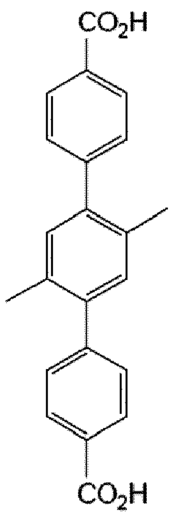
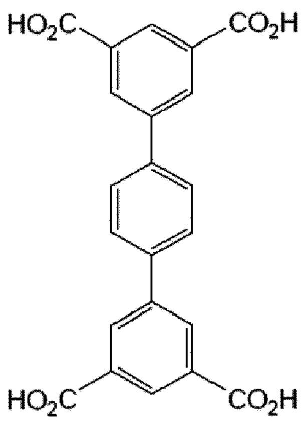
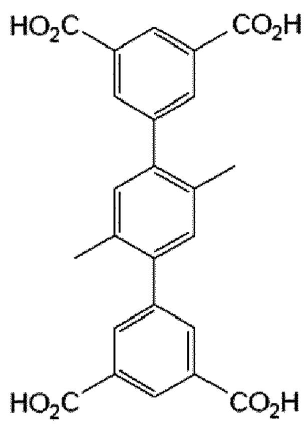

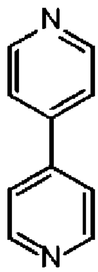
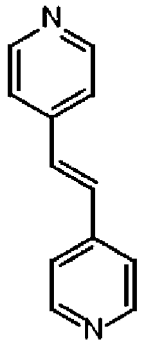
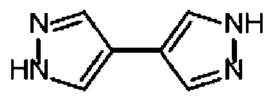
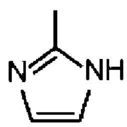

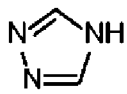
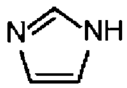
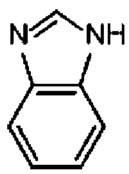
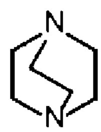
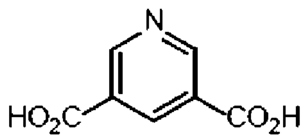

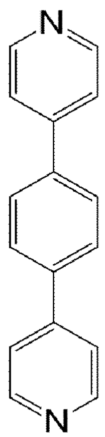
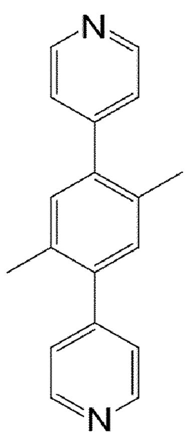
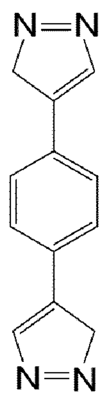

One alone or two or more kinds of these organic ligands may be used either singly or as combined.

The content of the organic ligand in the metal organic framework is preferably 1 to 6 relative to the molar number of the metal ion, more preferably 1 o 3, even more preferably 1 or 2.

The metal organic framework may contain any other component than a metal ion and a crosslinking organic ligand. Such components include a particle size regulator, a counter ion, a protein, a peptide and a DNA.

In the present invention, a covalent-bonding organic framework, an inorganic porous material and an organic porous material can also be used as a porous material, in addition to the above-mentioned metal organic framework. The covalent-bonding organic framework is a crystalline or amorphous porous polymer framework in which light atoms such as a hydrogen atom, a boron atom, a carbon atom and an oxygen atom bond via covalent bonding to form a network periodic framework. The covalent-bonding organic framework also has a hard crystalline skeleton and has a small nano-level pore size, and therefore secures a long spin-lattice relaxation time, and facilitates introduction of a polarization source or a polarization target into the pores thereof to keep them in a dispersed state. As the inorganic porous material, silica, organosilica, alumina, carbon and metal oxides are usable, and as the organic porous material, a fiber aggregate formed by entangling a fibrous polymer can be used.

(Condition of Pores of Porous Material)

The pore size of the pores of the porous material for use in the present invention is preferably 0.2 to 1000 nm, more preferably 0.2 to 100 nm, even more preferably 0.2 to 2 nm.
The pore size of the pores of the porous material can be measured by gas adsorption.
The porosity of the porous material is preferably 1% to 90%, more preferably 20% to 90%, even more preferably 40% to 90%.
The porosity of the porous material can be measured by gas adsorption.
Having a pore size and a porosity each falling within the above range, the porous material can introduce molecules of a polarization source and a polarization target into the pores of the porous material easily and in a sufficient amount, and can keep them in a well dispersed state.

[Other Components]

The composition of the present invention may be composed of a porous material and a polarization source alone, but may contain any other component. For example, in the case where the molecules constituting the polarization source has an acid group, preferably, a basic substance such as sodium hydroxide or potassium hydroxide is added to the composition. With that, protons can be ionized from the acid group of the polarization source to form a conjugated base to undergo interaction with the metal ion of a metal organic network. Accordingly, the amount of the polarization source to be introduced into the metal organic network can be significantly increased.
The composition of the present invention may contain a polarization target such as a protein, a peptide or a DNA, and a solvent molecule and a template molecule as other components. Here, the polarization target is a substance capable of transitioning nuclear spin polarization formed in a polarization source and a porous material. Regarding the description, preferred range and specific examples of the polarization target, reference may be made to the corresponding description in the section of "Hyperpolarization Method".

[Polarization Source Introduction Amount]

In the composition of the present invention, the amount of the polarization source to be introduced into the porous material is preferably 0.001 mol% to 1 mol% relative to the molar number of the metal ion of the metal organic framework therein, more preferably 0.001 mol% to 0.1 mol%, even more preferably 0.01 mol% to 0.05 mol%.
The amount of the polarization source to be introduced into the porous material can be determined by decomposing the composition with hydrochloric acid or the like to take out the polarization source therefrom, followed by measuring the peak intensity of the photoabsorption spectrum thereof.

[Spin-Lattice Relaxation Time $T_1$]

The spin-lattice relaxation time $T_1$ with the composition of the present invention is preferably 1 second or more, more preferably 2.5 seconds or more, even more preferably 10 seconds or more, further more preferably 60 seconds or more. With that, nuclear spin polarization can more readily accumulate, and therefore nuclear spin polarization of the composition and also nuclear spin of the polarization target can be thereby hyperpolarized.
The spin-lattice relaxation time $T_1$ can be determined by saturation recovery.

<Composition for Dynamic Nuclear Polarization>

Next, the composition for dynamic nuclear polarization of the present invention is described.
The composition for dynamic nuclear polarization of the present invention is characterized by containing the composition of the present invention.

Regarding the description, preferred range and specific examples of the composition of the present invention, reference may be made to the description of the section of "Composition" given hereinabove.

As described above, the molecule represented by the general formula (1) for use in the polarization source in the present invention is highly resistant to oxygen, and therefore can stably maintain the structure thereof even in air, and can surely exhibit the function as a polarization source. In addition, the composition of the present invention contains a porous material, and therefore secures a long spin-lattice relaxation time and facilitates introduction of a polarization source and a polarization target thereinto, and accordingly can hyperpolarize the nuclear spin thereof and the nuclear spin of the polarization target introduced into the composition. Consequently, the composition of the present invention can be effectively used as a composition for dynamic nuclear polarization.

<Hyperpolarized Composition>

Next, the hyperpolarized composition of the present invention is described.

The hyperpolarized composition of the present invention is one produced by hyperpolarizing the composition of the present invention.

Regarding the description, preferred range and specific examples of the composition of the present invention, and regarding the method and mechanism of hyperpolarization of the composition, reference may be made to the description of the section of <Composition> given hereinabove.

As described above, the composition of the present invention secures a long spin-lattice relaxation time $T_1$, and therefore by dynamic nuclear polarization, the nuclear spin thereof can be wholly hyperpolarized. Consequently, the hyperpolarized composition of the present invention has a high nuclear spin polarization ratio as a whole. Therefore, by bringing the hyperpolarized composition into contact with a polarization target, the nuclear spin polarization can transition to the polarization target to thereby hyperpolarize the nuclear spin of the polarization target.

The "hyperpolarized composition" of the present invention can be identified by detection of a peak having a higher intensity (a sensitized peak) in the NMR spectrum thereof than in the NMR spectrum of the composition in a thermal equilibrium state. The sensitized peak intensity of the hyperpolarized composition is preferably 10 times or more relative to the peak intensity corresponding to the composition in a thermal equilibrium state, more preferably 100 times or more, even more preferably 1000 times or more.

<Hyperpolarization Method>

Next, the hyperpolarization method of the present invention is described.

The hyperpolarization method of the present invention includes a step of contacting a substance with the hyperpolarized composition of the present invention, or a step of contacting a substance with the composition of the present invention followed by hyperpolarizing the composition to be a hyperpolarized composition.

In this description, the substance whose nuclear spin is polarized by the use of the above-mentioned composition for dynamic nuclear polarization (the substance targeted for polarization), and the substance whose nuclear spin is polarized by contacting it with the hyperpolarized composition of the present invention or with the composition of the present invention or by introducing it into the composition (the substance targeted for polarization) is referred to as "polarization target".

Regarding the description of the composition, reference may be made to the description of the section of "Composition" given hereinabove, and regarding the description of the hyperpolarized composition, reference may be made to the description of the section of "Hyperpolarized Composition" given hereinabove.

The hyperpolarization method of the present invention may further include a step of transitioning the nuclear spin polarization of the hyperpolarized composition to a polarization target.

In the absence of any additional step, the nuclear spin polarization of the hyperpolarized composition can be transitioned and diffused to the polarization target by contacting the polarization target with the hyperpolarized composition, but the case may include a step of inducing nuclear spin polarization transition. The method for the case includes a cross polarization method (CP method), a CP/MAS method using cross polarization, magic angle spinning and wideband decoupling as combined, and an adiabatic passage cross polarization method.

The nuclear spin polarization transition may be transition between the same nuclides like that from $^{1}H$ of a hyperpolarized composition to $^{1}H$ of a polarization target, or may be transition between different nuclides like transition between different nuclides from $^{1}H$ of a hyperpolarized composition to $^{13}C$ of a polarization target, or may also be both of these. Not specifically limited, the nuclide of the polarization target to which nuclear spin polarization is transitioned may be any other than that having a spin quantum number I of 0. Specific examples of the nuclide include $^{1}H$, $^{2}H$, $^{3}He$, $^{11}B$, $^{13}C$, $^{14}N$, $^{15}N$, $^{17}O$, $^{19}F$, $^{29}Si$, $^{31}P$, and $^{129}Xe$. As having a high natural abundance ratio, $^{1}H$, $^{14}N$, $^{19}F$, and $^{31}P$ are preferred, and as having a high NMR signal intensity, $^{1}H$ and $^{19}F$ are more preferred.

The polarization target to be contacted with the composition for dynamic nuclear polarization is preferably a

gas, a liquid or a solution. When such a polarization target is contacted with the hyperpolarized composition of the present invention, the polarization target can readily penetrate into the pores of a porous material to be included therein. With that, the nuclear spin polarization of the hyperpolarized composition can transition to the polarization target and the nuclear spin of the polarization target is thereby hyperpolarized.

The method of contacting such a gas, a liquid or a solution with the composition for dynamic nuclear polarization is not specifically limited. For example, such a polarization target may be injected into the composition, or as another method, a container is filled with such a polarization target and the composition may be arranged in the container so as to absorb the polarization target.

Alternatively, a polarization target is dissolved or dispersed in a liquid, and in that state, the resultant solution or dispersion may be mixed with the composition for dynamic nuclear polarization. At that time, the composition for dynamic nuclear polarization may directly polarize the polarization target or may polarize it via a liquid.

Here, the polarization target preferably contains at least one compound selected from hydrocarbons and hydrocarbon derivatives in which at least one hydrogen atom is substituted with a substituent.

The hydrocarbons may be acyclic compounds (aliphatic compounds) or cyclic compounds, may be saturated hydrocarbons or unsaturated hydrocarbons, and may be low-molecular compounds or high-molecular compounds. The cyclic compounds may be alicyclic or aromatic compounds. The carbon number of the hydrocarbon is not specifically limited, and is generally within a range of 1 to 10. A part of the carbon atoms in the molecule of the hydrocarbon may be substituted with a hetero atom. The hetero atom is not specifically limited, including N, P, O and S.

In the hydrocarbon derivatives, the substituent is not specifically limited, but preferably, at least one substituent therein is a substituent containing an atom of which the spin quantum number I is any other than 0, more preferably a substituent containing $^{13}C$, $^{15}N$, $^{19}F$, $^{29}Si$ or $^{31}P$, even more preferably a group containing $^{13}C$, or a fluorine atom.

A specific example of the polarization target is shown below. However, the polarization target for use in the hyperpolarization method of the present invention should not be limitatively interpreted by this specific example.

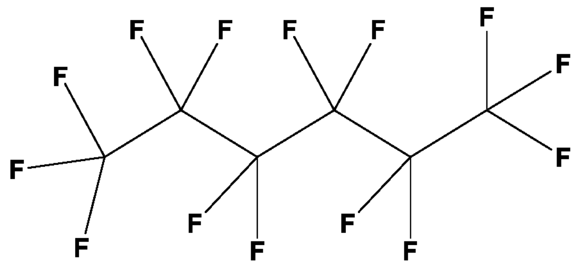

Bio-related materials are especially preferred for the polarization target. Here, "bio-related materials" include substances constituting a living body, and derivatives of substances constituting a living body. Examples of the substances constituting a living body include biopolymers (nucleic acids, proteins, polysaccharides), and constituent elements thereof of nucleotides, nucleosides, peptides, amino acids and saccharides, as well as lipids, vitamins, and hormones. Specific examples of the bio-related materials to be the polarization target include molecules as $^{13}C$-labeled in the red-circled portion in Fig. 2(A) in Chem. Soc. Rev., 2014, 43, 1627-1659, which is hereby incorporated in a part of this description by reference. Introducing such a molecule as a probe into a living body, MRI-scopy can be carried out using the present invention.

In the hyperpolarization method of the present invention, the amount of the polarization target to be introduced into the hyperpolarized composition is, in the case where the porous material in the composition is a metal organic framework, preferably 0.1 mol% to 500 mol% relative to the molar number of the metal ion therein, more preferably 1 mol% to 200 mol%, even more preferably 10 mol% go 100 mol%.

The amount of the polarization target to be introduced into the hyperpolarized composition can be determined by thermogravimetric analysis (TGA) or by NMR analysis after the metal organic framework has been dissolved in an acid.

<Hyperpolarized Substance>

Next, a hyperpolarized substance of the present invention is described.

The hyperpolarized substance of the present invention is a substance that has been hyperpolarized by the hyperpolarization method of the present invention. Regarding the description of the hyperpolarization method of the present invention, reference may be made to the description of the section of "Hyperpolarization Method" given hereinabove. Regarding the target substance to be hyperpolarized, reference may be made to the description, preferred range and specific examples described in the section of "Hyperpolarization Method" given hereinabove.

The polarization ratio of the hyperpolarized substance of the present invention is preferably $10^{-4}$ or more, more preferably $10^{-2}$ or more, even more preferably $10^{-1}$ or more.

The polarization ratio of a substance can be determined by comparing the NMR signal intensity after hyperpolarization and the signal intensity before hyperpolarization.

<NMR Measurement Method>

Next, an NMR measurement method of the present invention is described. The NMR measurement method of the present invention includes a step of measuring NMR (nuclear magnetic resonance) of a substance using the composition of the present invention. The NMR measurement method of the present invention also indicates a concept that includes an MRI method.

Regarding the description, preferred range and specific examples of the composition of the present invention, reference may be made to the section of "Composition" given hereinabove.

In the NMR measurement method of the present invention, an NMR measurement target is contacted with the hyperpolarized composition prepared by dynamic nuclear polarization of the composition of the present invention to hyperpolarize the nuclear spin of the measurement target, and then the NMR of the measurement target is measured according to a known NMR signal detection method. Alternatively, the NMR measurement target is introduced into the composition of the present invention, then the nuclear spin of the measurement target is hyperpolarized by dynamic nuclear polarization, and thereafter the NMR of the measurement target is measured according to a known NMR signal detection method. In the case where the nuclide to be measured by NMR is $^{13}C$ or $^{19}F$, hyperpolarized $^{1}H$ is further polarized into $^{13}C$ or $^{19}F$, and then the NMR thereof is measured.

Regarding the method of introducing the NMR measurement target into the composition of the present invention and the step of hyperpolarization, reference may be made to the description of the section of <Hyperpolarization Method> and <Composition> given hereinabove.

NMR signals can be detected according to a known method of a continuous-wave method or a pulse Fourier transformation method, and for example, for detection of NMR signals according to a pulse Fourier transformation method, an apparatus equipped with an RF coil (probe) and an amplifier can be used.

In the present invention, the nuclear spin of the measurement target is hyperpolarized using the composition of the present invention, and therefore the NMR signal can be detected at high intensity from the measurement target. Consequently, by applying the NMR measurement method, analysis of structures and physical properties of compounds by NMR spectrometry and checkup of biological organs can be carried out at high sensitivity.

<Other Embodiments of Polarization Source of Dynamic Nuclear Polarization>

Another embodiment of the polarization source of dynamic nuclear polarization is a polarization source of dynamic nuclear polarization containing a molecule having an oligoalkyleneoxy structure.

Still another embodiment of the polarization source of dynamic nuclear polarization is a dynamic nuclear polarization source having an ion pair that contains an ammonium ion having an oligoalkyleneoxy structure.

The oligoalkyleneoxy structure-having molecule has an extremely high dispersibility in an aqueous medium, and when excited in an aqueous medium, the polarization of the triplet electron spin of the molecule can be efficiently transitioned to the proton spin of a water molecule. Consequently, these molecules can be effectively used as a polarization source of dynamic nuclear polarization for NMR analysis of living bodies and biological materials.

Regarding the description, preferred range and specific examples of the oligoalkyleneoxy structure and the oligoalkyleneoxy structure-having ammonium ion, reference may be made to the description relating to "oligoalkyleneoxy structure" and "oligoalkyleneoxy structure-having ammonium ion" in R in [Molecule Represented by General Formula (1)] given hereinabove. Regarding the preferred range and specific examples of the anion ion to form an ion pair with the oligoalkyleneoxy structure-having ammonium ion, reference may be made to the description relating to "anion ion" in R in [Molecule Represented by General Formula (1)] given hereinabove. In particular, an anion group formed through proton ionization from a carboxy group (-COOH), a phosphono group ($-PO_3H_2$), a sulfo group ($-SO_3H$) or a hydroxy group (-OH) is preferred.

The molecule to be the polarization source is more preferably a molecule having an ion pair of an anion having a group $-COO^-$ and an oligoalkyleneoxy structure-having ammonium ion. In the molecule having an ion pair that contains an oligoalkyleneoxy structure-having ammonium ion, the number of the ion pairs existing in the molecule is 1 or more, preferably 2 or more, more preferably 3 or more, even more preferably 4 or more.

In the molecule having an oligoalkyleneoxy structure, and in the molecule having an ion pair that contains an oligoalkyleneoxy structure-having ammonium ion, the number of the oligoalkyleneoxy structures existing in the molecule is 1 or more, preferably 3 or more, more preferably 6 or more, even more preferably 9 or more, especially more preferably 12 or more.

<Method for increasing dispersibility in aqueous medium of polarization source>

Next, a method for increasing the dispersibility in an aqueous medium of a polarization source is described.

The method for increasing the dispersibility in an aqueous medium of a polarization source includes a step of introducing a substituent having an oligoalkyleneoxy structure into a molecule of a polarization source.

Also the method for increasing the dispersibility in an aqueous medium of a polarization source may be a method including a step of introducing a substituent that has an ion pair containing an oligoalkyleneoxy structure-having ammonium ion into a molecule of a polarization source, in place of the above-mentioned step.

When an oligoalkyleneoxy structure-containing group or a group containing an ion pair that contains an oligoalkyleneoxy structure-having ammonium ion is introduced into a molecule of a polarization source, the dispersibility in an aqueous medium of the polarization source molecule can be noticeably improved. The molecule of a polarization source whose dispersibility in an aqueous medium has been improved in the manner as above is, when excited in an aqueous medium, able to efficiently transition the polarization of the triplet electron spin thereof to the proton spin of the water molecule. The group containing an ion pair that contains an oligoalkyleneoxy structure-having ammonium ion to be introduced into the molecule of a polarization source is preferably a group having an ion pair of a group -COO" and an oligoalkyleneoxy structure-having ammonium ion.

Regarding the number of the ion pairs and the oligoalkyleneoxy structures to be introduced into the molecule in the above-mentioned step, reference may be made to the corresponding description in the section of <Other Embodiments of Polarization Source of Dynamic Nuclear Polarization> given hereinabove; and regarding the description, preferred range and specific examples of the oligoalkyleneoxy structure and the oligoalkyleneoxy structure-having ammonium ion, reference may be made to the description relating to "oligoalkyleneoxy structure" and "oligoalkyleneoxy structure-having ammonium ion" in R in [Molecule Represented by General Formula (1)] given hereinabove. The anion group to form an ion pair with the oligoalkyleneoxy structure-having ammonium may be any other than a group $-COO^-$. Regarding the preferred range and specific examples of the anion group, reference may be made to the description relating to "anion group" in R in the section of [Molecule Represented by General Formula (1)] given hereinabove.

<Dispersibility Improver in aqueous medium of polarization source molecule>

Next, a dispersibility improver in an aqueous medium of a polarization source molecule is described. The dispersibility improver contains a tertiary amine having an oligoalkyleneoxy group. When both a polarization source molecule and an oligoalkyleneoxy group-having tertiary amine are made to exist in an aqueous medium, the dispersibility in an aqueous medium of the polarization source molecule can be improved with the result that the polarization of the triplet electron spin of the polarization source molecule can be readily transitioned to the proton spin of the water molecule. Examples of the tertiary amine for use as the dispersibility improver include a compound represented by $NR^{21}_3$ (where $R^{21}$ represents a substituent, and at least one $R^{21}$ represents a group having an oligoalkyleneoxy group; and three $R^{21}$'S may be the same as or different from each other). One to three of three $R^{21}$'S may be an oligoalkyleneoxy group-having group, but preferably, all $R^{21}$'s are oligoalkyleneoxy group-having groups. Regarding the description, preferred range and specific examples of the oligoalkyleneoxy group, reference may be made to the description relating to "oligoalkyleneoxy group" in the section of [Molecule Represented by General Formula (1)] given hereinabove. When one or two of $R^{21}$'s are oligoalkyleneoxy groups, the remaining $R^{21}$' is any other substituent than an oligoalkyleneoxy group. Regarding the preferred range and specific examples of the substituent, for example, reference may be made to those exemplified as the substituent for R in the general formula (1).

Specific examples of the tertiary amine having an oligoalkyleneoxy group include tertiary amines among the specific examples of the oligoalkyleneoxy structure-having amine for use in forming an ammonium ion described hereinabove.

The polarization source molecule to which the dispersibility improver is applied includes a polarization source molecule having an acid group or a hydroxy group. Examples of the acid group in the polarization source include a carboxy group (-COOH), a phosphono group ($-PO_3H_2$), and a sulfo group ($-SO_3H$). Above all, a carboxy group is preferred. These polarization source molecules undergo acid base reaction with a tertiary amine having an oligoalkyleneoxy group to form an ion pair of an anion and an oligoalkyleneoxy group-having ammonium, in which the oligoalkyleneoxy group acts to improve the dispersibility in an aqueous medium of a polarization source molecule.

Examples

The characteristic features of the present invention are described more specifically with reference to Examples given below, in which the materials used, the details of the treatment and the treatment process may be appropriately modified or changed not overstepping the spirit and the scope of the invention. Accordingly, the invention should not be limitatively interpreted by the Examples mentioned below. For light absorption spectrometry, a spectrophotometer (V-670, V-770, by Japan Laser Corporation) was used. For NMR signal measurement, an NMR apparatus (JNM-ECA400, by JEOL Ltd.) was used. For time-resolved ESR spectrometry and dynamic nuclear polarization, a 532-nm pulse laser, an electromagnet, a microwave generator (HMC-T2220, by Analog Devices, Inc.) and an ESR detector were used as combined.

(Synthesis Example 1) Synthesis of precursor of compound 3

A precursor of a compound 3 was synthesized according to the following reaction scheme. Here, "a precursor of a compound 3" is a compound before forming an ion pair with an ammonium ion. In the following formula, Me represents a methyl group, and Bpin represents a 4,4,5,5-tetramethyl-1,3,2-dioxaborolan-2-yl group.

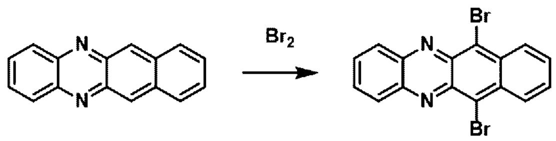


Intermediate 1

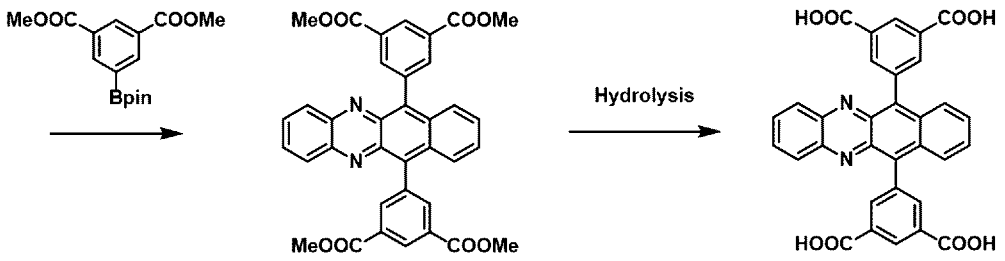


Intermediate 2

Precursor of Compound 3

Benzo[b]phenazine (1.0 g, 4.3 mmol) was put into a 300-mL flask, dissolved in chloroform (175 mL), and then cooled in an ice bath. Bromine (0.5 mL) was gradually added to the solution, and the resultant dark solution was stirred to be at room temperature. The reaction solution was washed with an aqueous sodium sulfite solution to remove the unreacted bromine. The organic layer was dried with anhydrous sodium sulfate, then filtered, and the filtrate was concentrated under reduced pressure. The crude product was purified through column chromatography using a mixed solvent of dichloromethane/hexane = 1/3 as an eluent to give a dark purple solid of 6,11-dibromobenzo[b]phenazine (intermediate 1) at a yield of 50%.
$^1$H-NMR (400 MHz, $CDCl_3$): σ (ppm) = 8.70-8.73 (m, 2H), 8.36-8.39 (m, 2H), 7.88-7.90 (m, 2H), 7.67-7.70 (m, 2H).

The intermediate 1 (388 mg, 1.0 mmol), dimethyl 5-(4,4,5,5-tetramethyl-1,3,2-dioxaborolan-2-yl)isophthalate (1.25 g, 4.0 mmol), PEPPSI-IPr(by Sigma Aldrich Corp.) (13.6 mg, 0.02 mmol) and cesium carbonate (1.3 g, 4.0 mmol) were dispersed in a mixed solvent of toluene (50 mL) and ethanol (10 mL) and refluxed for 8 hours in a nitrogen atmosphere. The reaction liquid was extracted with a mixed solvent of dichloromethane and brine, and the organic layer was dried with anhydrous sodium sulfate and concentrated under reduced pressure. The resultant crude product was purified through column chromatography using a mixed solvent of chloroform/methanol = 100/1 as an eluent to give a red solid of tetramethyl 5,5'-(benzo[b]phenazine-6,11-diyl)diisophthalate (intermediate 2).

The intermediate 2 (400 mg, 0.65 mmol) was suspended in a mixed solvent of tetrahydrofuran (50 mL), methanol (50 mL) and an aqueous 4 N sodium hydroxide solution (25 mL), and stirred overnight. 4 N hydrochloric acid was added to the reaction liquid to acidify it, then this was centrifuged to collect a precipitate, and the precipitate was washed a few times with methanol. The resultant filtrate was recrystallized with tetrahydrofuran added thereto, and taken out through filtration to give a red solid of a precursor of a compound 3 (5,5'-(benzo[b]phenazine-6,11-diyl)diisophthalate at a total yield of 15%.
$^1$H-NMR (400 MHz, DMSO-$d_6$): σ (ppm) = 13.4 (br, 4H), 8.72-8.73 (t, 2H), 8.35 (m, 4H), 7.98-8.01 (dd, 2H), 7.82-7.87 (m, 4H), 7.60-7.62 (dd, 2H).
Elemental analysis for $C_{32}H_{18}N_2O_8$: calculated (%) H 3.25, C 68.82, N 5.02; found (%) H 3.44, C 68.67, N 4.95.

[1-1] Evaluation of Oxygen Resistance of Polarization Source

The compound 1, the compound 2, pentacene and tetracene were each individually mixed with 1 mM tetrahydrofuran (polar solvent) to prepare liquid samples. Here, the compound 1 and pentacene are poorly soluble in tetrahydrofuran, and therefore their liquid samples were suspensions. The compound 2 and tetracene were individually formed into solutions as liquid samples.

Thus prepared, each liquid sample was put in air under ambient light, and the absorption spectrum thereof was measured at regular time intervals. The optical density of the absorption peak on the longest wavelength side was plotted on a graph with the horizontal axis indicating time to investigate the time dependence thereof. Here, the absorption peak position on the longest wavelength side was 640 nm for the suspension of the compound 1, 500 nm for the solution of the compound 2, 575 nm for the suspension of pentacene, and 473.5 nm for the solution of tetracene. Fig. 2 shows the time dependency of the optical density of each suspension of the compound 1 and pentacene; and Fig. 3 shows the time dependency of the optical density of each solution of the compound 2 and tetracene.

As shown in Figs. 2 and 3, reduction in the optical density of the compound 1 and the compound 2 was obviously smaller than that of pentacene and tetracene, that is, the temporal decomposition of the former two was suppressed.

This confirms that introduction of a nitrogen atom into the acene structure noticeably improved stability to oxygen.

[1-2] Production of polarization source-doped p-terphenyl crystal

Here, a p-terphenyl crystal was doped with a compound to be a polarization source to prepare samples for evaluation.

(Example 1) Production of p-terphenyl crystal doped with compound 1

The compound 1 was dispersed in dichloromethane and cast into a mortar, then dichloromethane was evaporated away, and p-terphenyl was added thereto and crushed. The resultant powder was put into an ampule and sealed up in vacuum, and melted by heating it in an oil bath at higher than 220°C (melting point of p-terphenyl). Subsequently, the ampule was immersed in liquid nitrogen to rapidly cool the melt to give a p-terphenyl crystal doped with 0.05 mol% of the compound 1, which was then taken out from the ampule to a mortar.

(Example 2) Production of p-terphenyl crystal doped with compound 2

A p-terphenyl crystal doped with 0.05 mol% of the compound 2 was produced in the same manner as in Example 1, except that the compound 2 was used in place of the compound 1.

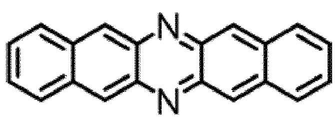

Compound 1

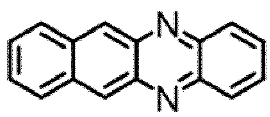

Compound 2

(Comparative Example 1) Production of p-terphenyl crystal doped with pentacene

A p-terphenyl crystal doped with 0.05 mol% pentacene was produced in the same manner as in Example 1, except that pentacene was used in place of the compound 1.

[1-3] Formation of spin-polarized triplet electron and evaluation of nuclear spin polarization enhancing effect by dynamic nuclear polarization

The p-terphenyl crystal doped with each compound was irradiated with excitation light at room temperature so as to excite the molecule of each compound, and immediately thereafter, the time-resolved ESR (electron spin relaxation) was measured. The results are shown in Fig. 4. Here, 532-nm pulse laser was used as the excitation light. In Fig. 4, "abs" on the vertical axis indicates microwave absorption, and "em" indicates microwave emission. In Fig. 4, a simulation curve sim of the ESR spectrum, as obtained by calculation with an EasySpin toolbox (MATLAB), is also shown.

In Fig. 4, the emission peak (peak protruding in the em direction) corresponds to $T_{1,0} \rightarrow T_{1,-1}$ transition (transition from m = 0 to m = -1 in $T_1$ state), and the absorption peak (peak protruding in the abs direction) corresponds to $T_{1,0} \rightarrow T_{1,+1}$ transition (transition from m = 0 to m = +1 in $T_1$ state) (see Fig. 1). The ESR spectra of the p-terphenyl crystals doped

with the compound 1 or 2 or pentacene all gave such emission peaks and absorption peaks, from which it is confirmed that these compounds produced triplet electron spin polarization. In addition, from the fitting parameter, it is confirmed that the triplet electron spin polarization of the compound 1 and the compound 2 is on the same level as that of pentacene, that is, the triplet electron spin polarization is not influenced by aza-substitution.

Fig. 5 shows a temporal change of the ESR peak of the p-terphenyl crystal doped with any of those compounds. In Fig. 5, "pos→" on the vertical axis indicates positive polarization; and "neg→" indicates negative polarization. An approximating expression of the curve in Fig. 5 is represented by the following formula (I), and the numerical values of the parameters in the formula (I) are shown in Table 1.

$$A \exp(-t/\tau_A) + B \exp(-t/\tau_B) + C \qquad \cdots (I)$$

| Compound | A | $\tau_A$(μs) | B | $\tau_B$(μs) |
|---|---|---|---|---|
| Pentacene | 0.23 | 18 | -0.033 | 140 |
| Compound 1 | 0.52 | 3.1 | -0.038 | 26 |
| Compound 2 | 0.22 | 11 | -0.023 | 98 |

Regarding the negative polarization of the ESR peak in Fig. 5, the occupancy rate of the Ti,+i component became larger than that of the $T_{1,0}$ component by temporal change. As shown in Table 1, $\tau_B$ (transition time of $T_1 \rightarrow S_0$) of the ESR peak of the compounds 1 and 2 was shorter than that of pentacene.

Next, the nuclear spin polarization enhancing effect by dynamic nuclear polarization of each compound was evaluated by measurement of $^1$H NMR signals. Specifically, the p-terphenyl crystal doped with the compound 1 or 2 or pentacene was irradiated with 532-nm pulse laser so that the state of the molecule of each compound was transitioned toward an excited triplet state, and then with irradiation with microwaves, the external magnetic field was swept at around 0.676T for dynamic nuclear polarization. The process of the dynamic nuclear polarization was repeated, and during that process, $^1$H NMR signals were measured at regular intervals. The found $^1$H NMR signal (signal voltage) was substituted for $E_{DNP}$ in the following formula (II) to determine the enhancement factor ε. Fig. 6 shows a graph of the results as plotted therein (buildup curve of $^1$H spin polarization), in which the horizontal axis indicates a processing time. Here, as reference data, $^1$H NMR signal data of ethanol in a thermal equilibrium state in a magnetic field at 0.676 T at 300 K were used.

$$\varepsilon = \frac{N_{ref}}{N_{DNP}} \frac{T_{DNP}}{T_{ref}} \frac{g_{ref}}{g_{DNP}} \frac{E_{DNP}}{E_{ref}} \qquad \cdots (II)$$

In the formula (II), $N_{DNP}$ and $N_{ref}$ each represent a number of $^1$H spins, $T_{DNP}$ and $T_{ref}$ each represent a temperature, $g_{DNP}$ and $g_{ref}$ each represent a receiver gain, $E_{DNP}$ and $E_{ref}$ each represent a signal voltage. Among these, symbols with a subscript "DNP" are parameters for the compounds used as a polarization source, and symbols with a subscript "ref" are parameters for ethanol.

From the enhancement factor ε, a $^1$H spin polarization rate P was calculated using the following formula (III).

$$P = \varepsilon \tanh \frac{\gamma \hbar B}{2kT} \qquad \cdots (III)$$

In the formula (III), γ represents a magnetogyric ratio, h represents the Planck's constant, B represents a magnetic flux density, k represents the Boltzmann constant, and T represents a temperature.

From Fig. 4 and the calculation results by the formula (III), the final $^1$H spin polarization ratio of the compound 2 is 0.19%, the enhancement factor thereof is 840, and these are comparable to the $^1$H spin polarization ratio (0.21%) of pentacene and the enhancement factor (930) thereof. Further, the final $^1$H spin polarization ratio of the compound 1 is 0.22%, the enhancement factor thereof is 970, and these are beyond the data of pentacene. In that manner, the reason why the compound 1 attained a high nuclear spin polarization enhancing effect is considered to be because the triplet lifetime of the compound 1 is 3.3 μs and is shorter than the triplet lifetime, 89 μs of pentacene, and therefore the former can effectively prevent the spin-lattice relaxation time from being shortened by the presence of a paramagnetic triplet electron.

From the above results, it is confirmed that the molecule having an azaacene skeleton with a nitrogen atom

introduced into an acene structure (molecule represented by the general formula (1)) is excellent in oxygen resistance and has a nuclear spin polarization enhancing effect on the same level or more than that of pentacene, and is therefore extremely useful as a polarizations source for dynamic nuclear polarization.

[2-1] Evaluation of Compound 3

(Example 3) Preparation of aqueous solution of compound 3

The precursor of the compound 3 (1 mM) and tris[2-(2-methoxyethoxy)ethyl]amine (MEEA) (4 mM) were dissolved in water and reacted to give an aqueous solution of the compound 3.

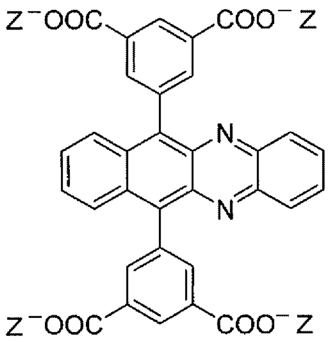

Z represents an ammonium ion represented by the following formula.

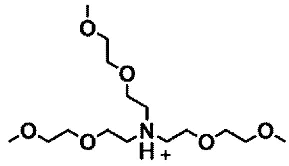

Compound 3

] An aqueous solution of the compound 3 frozen with liquid nitrogen (crystalline ice containing the compound 3), and a molecular dispersion of the compound 3 in a dimethyl sulfoxide solution also frozen with liquid nitrogen each were analyzed to measure the emission spectrum with 532-nm excitation light at 140 K. The crystalline ice containing the compound 3 gave emission peaks (557 nm, 596 nm) slightly on the blue side than the emission peaks from the frozen dimethyl sulfoxide solution of the compound 3 (560 nm, 609 nm). It is known that when molecules aggregate, the emission peak from the resultant aggregate may shift on the red side, and therefore, the blue shift in this case indicates that the molecules of the compound 3 are in a highly molecular-dispersed state in the crystalline ice. In addition, an aqueous solution of the following compound was also frozen like the aqueous solution of the compound 3, and evaluated in point of the dispersibility by analyzing the emission spectrum thereof. As a result, the dispersibility of the compound 3 was better than that of the following compound.

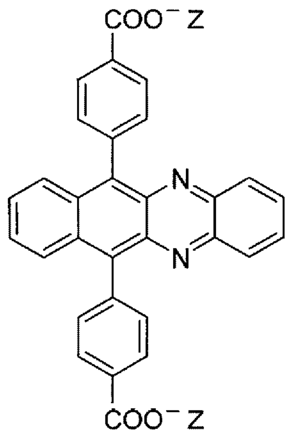

Z represents an ammonium ion represented by the following formula.

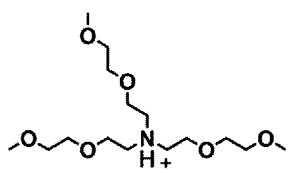

Next, an aqueous solution of the compound 3 was frozen with liquid nitrogen, then irradiated with 532 nm pulse laser excitation light at 140 K, and immediately thereafter, a time-resolved ESR thereof was measured. The results are shown in Fig. 7. In addition, the temporal change of the ESR peak observed at 354 mT is shown in Fig. 8. In Fig. 7, "Em.", "Abs." and "sim" have the same meanings as "abs", "em" and "sim", respectively, in Fig. 4; and in Fig. 8, "Pos.→" and "←Neg." are the same meanings as "pos→" and "←neg", respectively, in Fig. 5. As shown in Fig. 7, the ESR spectrum of the compound 3 gave an emission peak derived from $T_{1,0} \rightarrow T_{1,-1}$ transition, and an absorption peak derived

from $T_{1,0}{\rightarrow}T_{1,+1}$ transition, from which it is confirmed that the compound produces triplet electron spin polarization. The ESR spectrum of the compound has a typical spectral form often given by an unsubstituted benzo[b]phenazine, from which it is confirmed that the triplet electron spin polarization is not influenced by hydrophilic modification. The ESR peak lifetime (triplet lifetime) thereof was 37 μs, which was sufficient length for transitioning the polarization of triplet electron spin to nuclear spin.

Next, the nuclear spin polarization enhancing effect by dynamic nuclear polarization of each compound was evaluated by measuring the $^1$H NMR signals thereof. Specifically, an aqueous solution of the compound 3 was frozen with liquid nitrogen, then irradiated with 532 nm pulse laser (400 Hz) at 140 K so as to transition the molecule of the compound 3 to be in an excited triplet state, and thereafter with irradiation with microwaves (17.7 GHz), the external magnetic field was swept at around 664 ± 30 mT for dynamic nuclear polarization. The process of the dynamic nuclear polarization was repeated, and during that process, $^1$H NMR signals were measured at regular intervals. The found $^1$H NMR signal (signal voltage) was substituted for $E_{DNP}$ in the formula (II) to determine the enhancement factor ε. Fig. 9 shows a graph of the results as plotted therein (buildup curve of $^1$H spin polarization), in which the horizontal axis indicates a processing time. Here, as reference data for ε, $^1$H NMR signal data of ethanol in a thermal equilibrium state at 140 K were used. The broken line in Fig. 9 indicates a fitting curve obtained from the following formula (IV).

$$A[1-\exp(-t/T_B)] \qquad (IV)$$

In the formula (IV), t represents a processing time, and $T_B$ represents a spin lattice relaxation time.

As shown in Fig. 9, the buildup curve of the crystalline ice containing the compound 3 fits well to the fitting curve, and the spin lattice relaxation time $T_B$ is 11.6 minutes, and the enhancement factor ε is 23. From the results, it is confirmed that, by transitioning the polarization of the triplet electron spin of the compound 3 to the crystalline ice, the nuclear spin polarization of $^1$H of water molecules can be enhanced.

Industrial Applicability

The polarization source of the present invention has high oxygen resistance and can stably transition the spin polarization of the formed triplet electron to nuclei by dynamic nuclear polarization. Consequently, using the polarization source of the present invention, dynamic nuclear polarization can be effectively attained even in air. Therefore, the industrial applicability of the present invention is great.

## Claims

1. A polarization source for dynamic nuclear polarization containing a molecule represented by the following general formula (1):

General Formula (1)

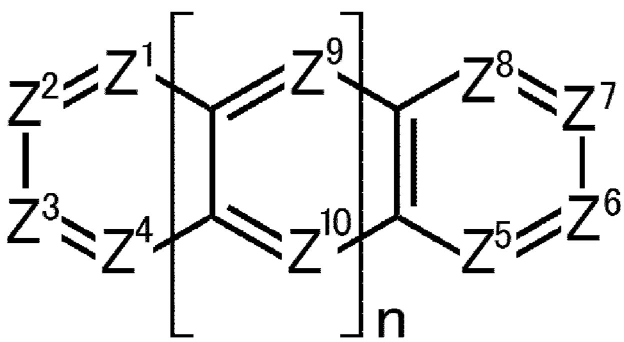

wherein one to six of $Z^1$ to $Z^{10}$ each represent N, and the others each independently represent C-R, in which R represents a hydrogen atom or a substituent, n represents an integer of 1 to 4, when n is 2 or more, plural $Z^9$'s may be the same or different, and plural $Z^{10}$'s may be the same or different.

2. The polarization source according to claim 1, wherein two to 2n (when n is 1, two) of $Z^1$ to $Z^{10}$ in the general formula (1) each represent N, and the others each independently represent C-R, in which R represents a hydrogen atom

or a substituent.

**3.** The polarization source according to claim 1 or 2, wherein n is 2 or 3.

**4.** The polarization source according to any one of claims 1 to 3, satisfying at least one of the following three requirements:

<1> $Z^1$ and $Z^4$ are both N,
<2> $Z^5$ and $Z^8$ are both N,
<3> $Z^9$ and $Z^{10}$ are both N.

**5.** The polarization source according to any one of claims 1 to 4, wherein at least one of plural R's in the general formula (1) is a substituent containing one or more atoms selected from the group consisting of a halogen atom, a nitrogen atom, an oxygen atom, a sulfur atom, a boron atom, a phosphorus atom and a silicon atom.

**6.** The polarization source according to claim 5, wherein at least one of $Z^1$, $Z^2$, $Z^7$, $Z^8$ and $Z^9$ is C-$R^{11}$ , at least one of $Z^3$ , $Z^4$, $Z^5$, $Z^6$ and $Z^{10}$ is C-$R^{12}$, and $R^{11}$ and $R^{12}$ each are independently a substituent containing one or more atoms selected from the group consisting of a halogen atom, a nitrogen atom, an oxygen atom, a sulfur atom, a boron atom, a phosphorus atom and a silicon atom.

**7.** The polarization source according to any one of claims 1 to 6, wherein at least one of plural R's in the general formula (1) contains one or more selected from the group consisting of a halogen atom, a cyano group, a carbonyl group, an ester group, a carboxy group, a sulfo group, a phosphono group, a phosphonoxy group, a boron atom, a phosphorus atom, a silicon atom, a hydroxy group, a thiol group, a benzene ring, a pyridine ring, a pyridazine ring, a pyrimidine ring, a pyrazine ring and an amino group.

**8.** The polarization source according to any one of claims 1 to 7, wherein at least one of plural R's in the general formula (1) is a group containing an oligoalkyleneoxy structure.

**9.** The polarization source according to any one of claims 1 to 8, wherein the molecule is represented by the following general formula (2):

General Formula (2)

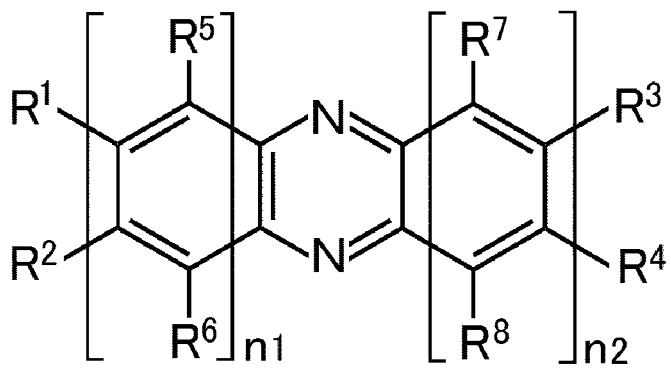

wherein $R^1$ to $R^8$ each independently represent a hydrogen atom or a substituent, n1 and n2 each independently represent an integer of 0 to 4, and n1 + n2 is an integer of 2 to 5, when n1 is 2 or more, plural $R^5$'s may be the same or different, and plural $R^6$'s may be the same or different, when n2 is 2 or more, plural $R^7$'S may be the same or different, and plural $R^8$'S may be the same or different.

**10.** A composition containing the polarization source of any one of claims 1 to 9.

**11.** The composition according to claim 10, containing a porous material and the polarization source.

**12.** The composition according to claim 11, wherein the porous material is a metal organic framework or a covalent-bonding organic framework.

**13.** The composition according to claim 12 containing a metal organic framework, wherein the organic ligand of the metal organic framework has a cyclic structure substituted with a substituent, and at least one hydrogen atom of the substituent is substituted with a deuterium atom.

**14.** The composition according to claim 12 containing a metal organic framework, wherein the organic ligand of the metal organic framework has an imidazole skeleton.

**15.** The composition according to any one of claims 12 to 14 containing a metal organic framework, wherein the metal ion of the metal organic framework contains a di-to tetra-valent metal ion.

**16.** The composition according to any one of claims 12 to 15 containing a metal organic framework, wherein the metal ion of the metal organic framework contains a zinc ion $Zn^{2+}$.

**17.** The composition according to any one of claims 12 to 16 containing a metal organic framework, wherein the molecule constituting the polarization source has a functional group that interacts with the metal ion of the metal organic framework.

**18.** The composition according to claim 17, wherein the functional group is an acid group.

**19.** The composition according to claim 17, wherein the functional group is a carboxy group or a carboxylate anion.

**20.** The composition according to any one of claims 11 to 19, wherein the molecule constituting the polarization source exists in the pores of the porous material.

**21.** The composition according to any one of claims 11 to 20, wherein the content of the polarization source is 0.01% by mol or more relative to the number of mols of the metal ion of the metal organic framework.

**22.** The composition according to any one of claims 11 to 21, wherein the spin-lattice relaxation time $T_1$ of the composition is 2.5 seconds or more.

**23.** The composition according to any one of claims 10 to 22, further containing a substance capable of transitioning the nuclear spin polarization formed by the polarization source and the porous material.

**24.** A composition for dynamic nuclear polarization containing the composition of any one of claims 10 to 23.

**25.** A hyperpolarized composition prepared by hyperpolarizing the composition of any one of claims 10 to 23.

**26.** A method for hyperpolarizing a substance, including a step of contacting a substance with the hyperpolarized composition of claim 25, or a step of contacting a substance with the composition of any one of claims 10 to 21, and then hyperpolarizing the composition to be a hyperpolarized composition.

**27.** The hyperpolarization method according to claim 26, wherein the substance is a liquid or a solution.

**28.** The hyperpolarization method according to claim 26, wherein the substance is a gas.

**29.** The hyperpolarization method according to claim 27 or 28, wherein the substance is contacted with the hyperpolarized composition or the composition by infiltrating the substance into the porous material that constitutes the hyperpolarized composition or the composition.

**30.** The hyperpolarization method according to any one of claims 26 to 29, wherein the substance contains at least one compound selected from a hydrocarbon and a derivative of a hydrocarbon in which at least one hydrogen atom is substituted with a substituent.

**31.** The hyperpolarization method according to claim 30, wherein the substance contains a derivative of a hydrocarbon in which at least one hydrogen atom is substituted with a substituent, and at least one substituent contains an atom of which the spin quantum number I is any other than 0.

**32.** The hyperpolarization method according to claim 30 or 31, wherein the substituent is a fluorine atom.

**33.** The hyperpolarization method according to any one of claims 26 to 32, including a step of transitioning the nuclear spin polarization of the hyperpolarized composition to the substance.

**34.** The hyperpolarization method according to claim 33, wherein the step of transitioning the nuclear spin polarization of the hyperpolarized compound to the substance is carried out by irradiating the hyperpolarized composition and the substance kept in contact with each other with microwaves.

**35.** A substance hyperpolarized according to the method of any one of claims 26 to 34.

**36.** An NMR measurement method including a step of measuring NMR of a substance using the composition of any one of claims 10 to 23.

[Fig. 1]

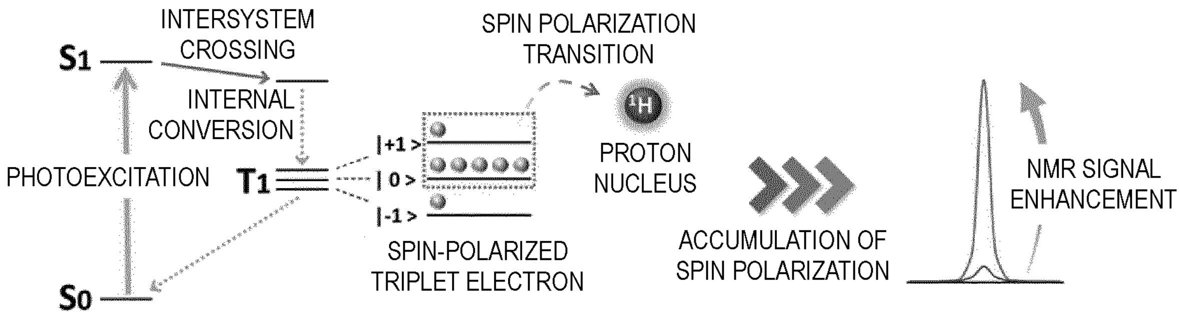


[Fig. 2]

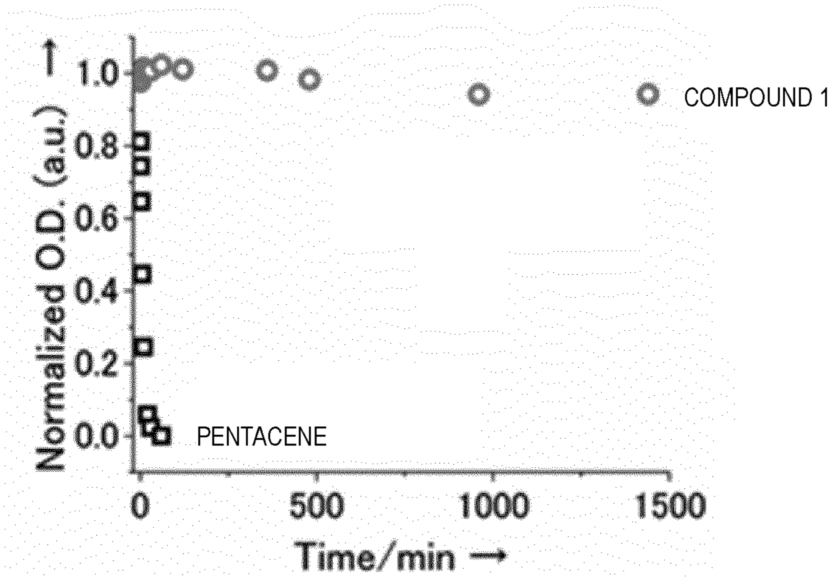


[Fig. 3]

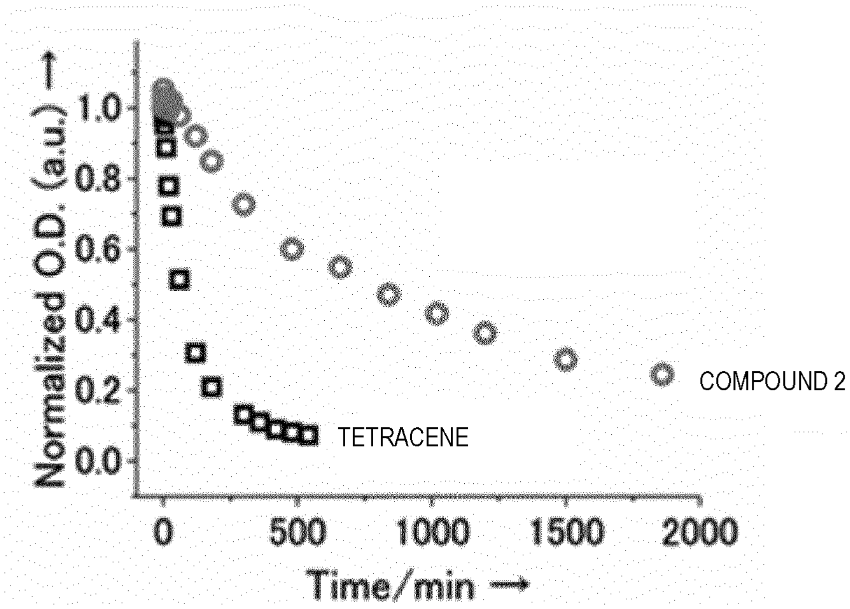

[Fig. 4]

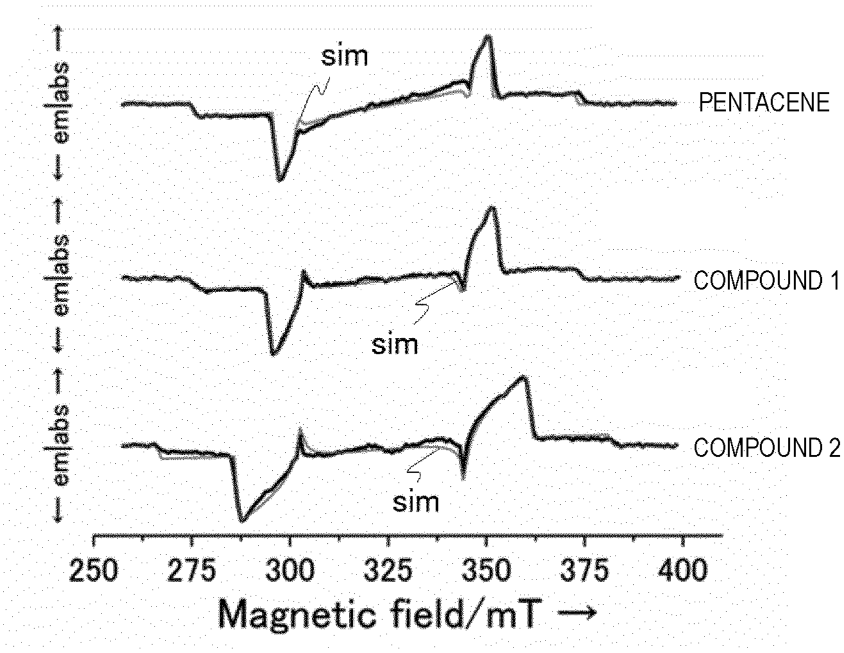
sim
PENTACENE
COMPOUND 1
sim
COMPOUND 2
sim
↓ em|abs ↑
↓ em|abs ↑
↓ em|abs ↑
250
275
300
325
350
375
400
Magnetic field/mT →

[Fig. 5]

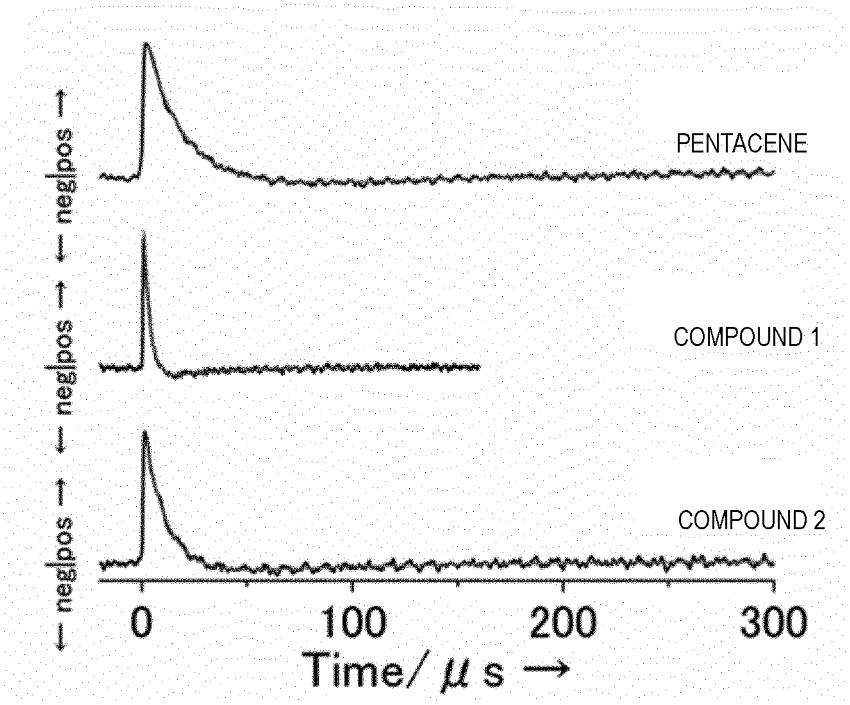
PENTACENE
COMPOUND 1
COMPOUND 2
↓ neg|pos ↑
↓ neg|pos ↑
↓ neg|pos ↑
0
100
200
300
Time/ μ s →

[Fig. 6]

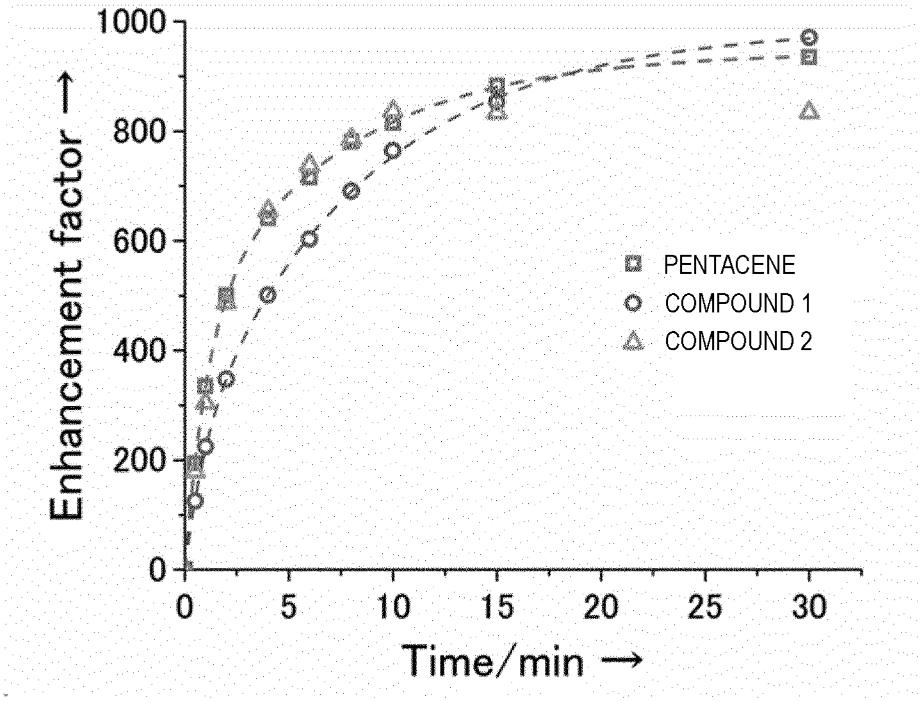
1000
800
600
400
200
0
Enhancement factor →
0
5
10
15
20
25
30
Time/min →
PENTACENE
COMPOUND 1
COMPOUND 2

[Fig. 7]

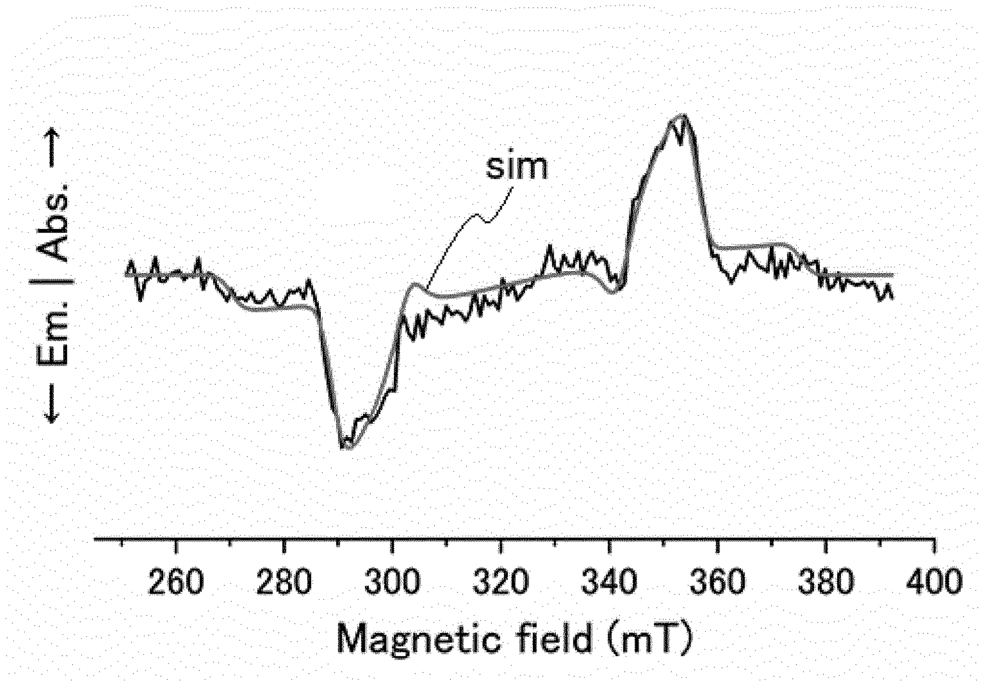
← Em. | Abs. →
sim
260
280
300
320
340
360
380
400
Magnetic field (mT)

[Fig. 8]

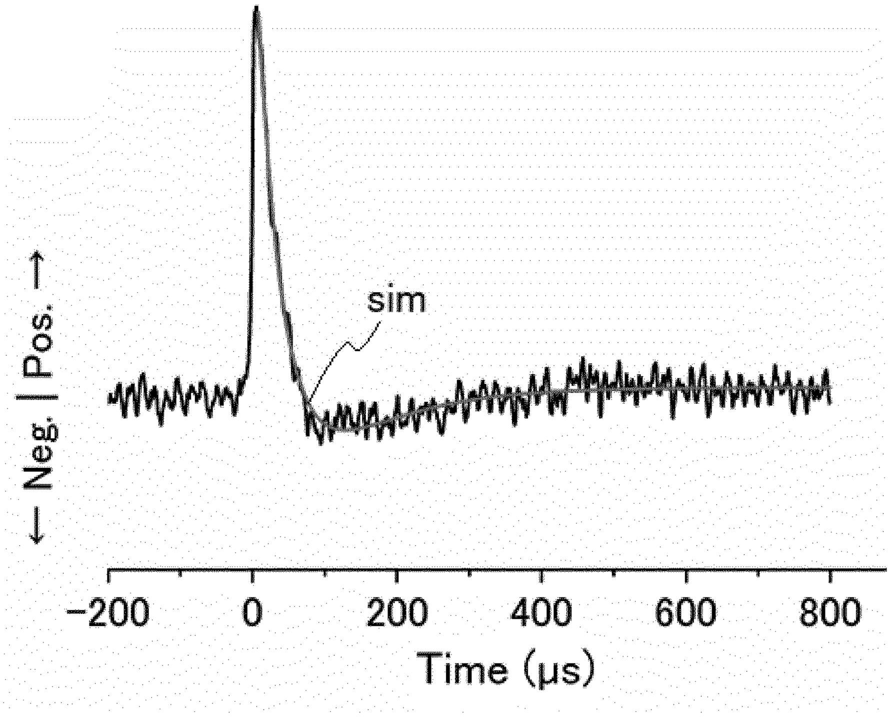
sim
← Neg. | Pos. →
−200
0
200
400
600
800
Time (μs)

[Fig. 9]

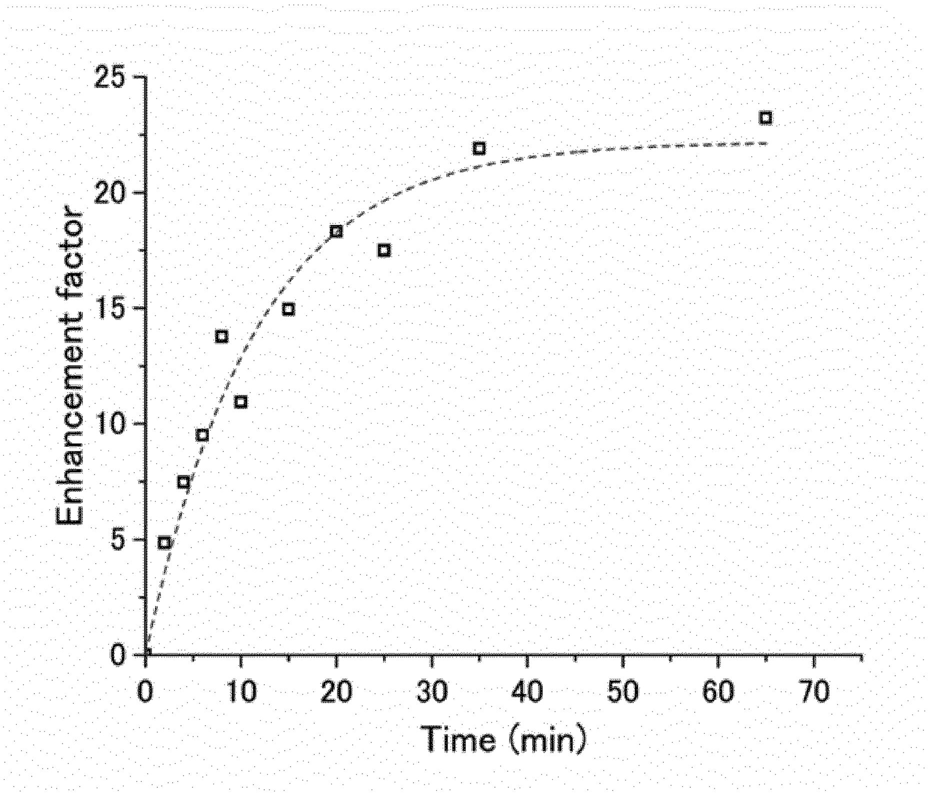
Enhancement factor
25
20
15
10
5
0
0
10
20
30
40
50
60
70
Time (min)

## INTERNATIONAL SEARCH REPORT

International application No.
PCT/JP2020/008482

A. CLASSIFICATION OF SUBJECT MATTER
C07D 241/36(2006.01)i; G01N 24/00(2006.01)i; G01N 24/12(2006.01)i
FI: G01N24/00 100B; C07D241/36; G01N24/12 510L

According to International Patent Classification (IPC) or to both national classification and IPC

B. FIELDS SEARCHED

Minimum documentation searched (classification system followed by classification symbols)
G01N24/00-24/ 14

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| Y | JP 2017-15443 A (RIKEN, INSTITUTE OF PHYSICAL AND CHEMICAL RESEARCH) 19.01.2017 (2017-01-19) paragraphs [0003], [0012]-[0013], [0033]-[0042] | 1-36 |
| Y | JP 2011-93827 A (SHARP CORP.) 12.05.2011 (2011-05-12) paragraphs [0004]-[0005], [0055]-[0058] | 1-36 |
| Y | WU, Judy I., "4n π Electrons but Stable: N,N-Dihydrodiazapentacenes", J. Org. Chem., 2009, vol. 74, pp. 4343-4349, column "abstract" | 1-36 |
| Y | FUJIWARA, Saiya, "Dynamic Nuclear Polarization of Metal-Organic Frameworks Using Photoexcited Triplet Electrons", J. Am. Chem. Soc., 07 November 2018, vol. 140, pp. 15606-15610, column "abstract" | 11-36 |

☒ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>13 May 2020 (13.05.2020) | Date of mailing of the international search report<br>02 June 2020 (02.06.2020) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2020/008482

C (Continuation). DOCUMENTS CONSIDERED TO BE RELEVANT

| Category* | Citation of document, with indication, where appropriate, of the relevant passages | Relevant to claim No. |
|---|---|---|
| Y | ナノ多孔性材料を室温で高核偏極化することに世界で初めて成功～生体分子の高感度 MRI 観測へ新たな道～, 九州大学プレスリリース［オンライン], 08 November 2018, http://kyushu-u.ac.jp/f/34490/18_11_08_1.pdf, [retrieval date 13 May 2020], entire text, non-official translation ("New Method for Enhancing the Sensitivity of NMR/MRI at Room Temperature- The new path to highly sensitive MRI observation of biomolecules-", Kyushu University Press Release[online]) | 11-36 |
| A | JP 2002-124384 A (FUJITSU LTD.) 26.04.2002 (2002-04-26) entire text, all drawings | 1-36 |
| A | TANG, Qin, "A Meaningful Analogue of Pentacene: Charge Transport, Polymorphs, and Electronic Structures of Dihydrodiazapentacene", Chem. Mater., 2009, vol. 21, pp. 1400-1405 entire text | 1-36 |
| P, X | KOUNO, Hironori, "Nonpentacene Polarizing Agents with Improved Air Stability for Triplet Dynamic Nuclear Polarization at Room Temperature", J. Phys. Chem. Lett., 01 April 2019, vol. 10, pp. 2208-2213 entire text | 1-36 |

Form PCT/ISA/210 (continuation of second sheet) (January 2015)

**INTERNATIONAL SEARCH REPORT**
Information on patent family members

International application No.
PCT/JP2020/008482

| Patent Documents referred in the Report | Publication Date | Patent Family | Publication Date |
|---|---|---|---|
| JP 2017-15443 A | 19 Jan. 2017 | US 2018/0188338 A1 paragraphs [0005]-[0008], [0036]-[0062], [0122]-[0161]<br>WO 2017/002761 A1<br>EP 3315987 A1 | |
| JP 2011-93827 A | 12 May 2011 | (Family: none) | |
| JP 2002-124384 A | 26 Apr. 2002 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017015443 A


### Non-patent literature cited in the description


- *Chem. Soc. Rev.,* 2014, vol. 43, 1627-1659